(19) Europäisches Patentamt
European Patent Office
Office européen des brevets

(11) Numéro de publication : **0 286 523 B1**

# (12) FASCICULE DE BREVET EUROPEEN

(45) Date de publication du fascicule du brevet : **22.01.92 Bulletin 92/04**

(51) Int. Cl.$^5$ : **C07C 59/86,** C07C 59/90, C07C 59/54, C07C 57/50

(21) Numéro de dépôt : **88400796.4**

(22) Date de dépôt : **01.04.88**

## (54) Nouveaux dérivés aromatiques d'acide butyrique, leur procédé de préparation et leur utilisation en cosmétique ainsi qu'en médecine humaine et vétérinaire.

(30) Priorité : **03.04.87 FR 8704711**

(43) Date de publication de la demande : **12.10.88 Bulletin 88/41**

(45) Mention de la délivrance du brevet : **22.01.92 Bulletin 92/04**

(84) Etats contractants désignés : **AT BE CH DE ES FR GB GR IT LI NL SE**

(56) Documents cités :
**CH-A- 610 875**
**FR-A- 1 566 213**
**FR-A- 2 300 551**

(73) Titulaire : **L'OREAL**
**14, Rue Royale**
**F-75008 Paris (FR)**

(72) Inventeur : **Maignan, Jean**
**8, rue Halévy**
**F-93290 Tremblay Les Gonesse (FR)**
Inventeur : **Malle, Gérard**
**18, Grande Rue**
**F-77580 Villiers Sur Morin (FR)**
Inventeur : **Lang, Gérard**
**44 avenue Lacour**
**F-95210 Saint Gratien (FR)**

(74) Mandataire : **Nony, Michel et al**
**Cabinet NONY & CIE, 29, rue Cambacérès**
**F-75008 Paris (FR)**

EP 0 286 523 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés aromatiques d'acide butyrique, leur procédé de préparation et leur utilisation en cosmétique ainsi qu'en médecine humaine et vétérinaire.

L'état de la technique concernant les composés selon l'invention est essentiellement représenté par les demandes de brevet français n° 1.566.213 et 2.300.551 ainsi que par le brevet suisse n° 610.875.

Les composés selon l'invention, de par leur activité inhibitrice de la synthèse des lipides, sont d'un grand intérêt en cosmétique dans le traitement du cuir chevelu et de la peau présentant un aspect gras.

Ces nouveaux dérivés aromatiques d'acide butyrique selon l'invention peuvent être représentés par la formule générale suivante :

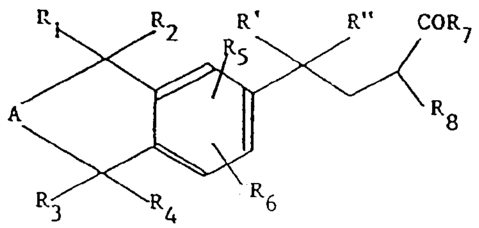

(I)

dans laquelle:

$R_1$, $R_2$, $R_3$ et $R_4$ représentent, indépendamment l'un de l'autre, un atome d'hydrogène ou un radical alkyle inférieur, au moins deux des radicaux $R_1$ à $R_4$ étant différents d'un acore d'hydrogène,

A représente un radical méthylène ou diméthylène, substitué ou non par un radical alkyle inférieur ; lorsque A représente un radical diméthylène, $R_1$ et $R_3$ peuvent former ensemble un radical méthylène ou diméthylène,

$R_5$ et $R_6$ représentent un atome d'hydrogène, un atome d'halogène, un radical alkyle inférieur, un radical alekoxy inférieur ou un radical hydroxyle,

R′ représente un atome d'hydrogène, un radical hydroxyle, un radical alkoxy inférieur ou un radical acyloxy en $C_1$-$C_4$,

R″ représente un atome d'hydrogène ou un radical alkoxy inférieur, ou R′ et R″, pris ensemble, forment un radical oxo (=O) ou hydroxyimino (=N-OH),

$R_8$ représente un atome d'hydrogène ou un radical alkyle inférieur, et $R_7$ représente le radical -$OR_9$ ou

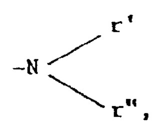

$R_9$ représentant un atome d'hydrogène, un radical alkyle ayant de 1 à 20 atomes de carbone, un radical monohydroxyalkyle, polyhydroxyalkyle, aryle ou aralkyle, un reste de sucre, un radical carboxyalkyle ou alkoxycarbonylalkyle,

r′ et r″ représentant un atome d'hydrogène, un radical alkyle inférieur, un radical monohydroxyalkyle éventuellement interrompu par un hétéroatome, un radical monohydroxyalkyle, un radical ou benzyle, un reste d'amino-acide ou de sucre aminé ou, pris ensemble, forment un hétérocycle éventuellement substitué par radical hydroxyalkyle en $C_1$-$C_3$, et les sels desdits composés de formule (I) et leurs isomères optiques ainsi que les formes tautomères des composés de formule (I) (en particulier les lactones et lactames correspondants).

Par radical alkyle inférieur, on doit entendre un radical ayant de 1 à 6 atomes de carbone.

Par radical alkyle inférieur ou ayant jusqu'à 20 atomes de carbone, on doit entendre notamment les radicaux méthyle, éthyle, propyle, isopropyle, butyle, tertiobutyle, éthyl-2 hexyle, octyle, dodécyle, hexadécyle et octadécyle.

Par radical monohydroxyalkyle, on doit entendre un radical ayant de 2 à 6 atomes de carbone, notamment un radical hydroxy-2 éthyle, hydroxy-2 propyle ou hydroxy-2 éthoxyéthyle,

Par radical polyhydroxyalkyle, on doit entendre un radical contenant de 3 à 6 atomes de carbone et de 2 à 5 groupes hydroxyles tels que les radicaux dihydroxy-2,3 propyle, dihydroxy-1,3 propyl-2 ou le reste du pentaérythritol.

Parmi les radicaux alkoxy inférieurs, on peut notamment citer les radicaux méthoxy, isopropoxy, butoxy et tert-butoxy.

Par reste d'un sucre, on doit entendre un reste dérivant du glucose, du mannose, de l'érythrose ou du galactose.

Parmi les restes de sucre aminé, on peut citer ceux dérivant de glucosamine, de galactosamine, de mannosamine ou de la méglumine.

Lorsque les radicaux r′ et r″, pris ensemble, forment avec l'atome d'azote auquel ils sont reliés, un hétérocycle, celui-ci est de préférence un radical pipéridino, pipérazino, morpholino, pyrrolidino ou (hydroxy-2 éthyl)-4 pipérazino.

Lorsque les composés selon l'invention se présentent sous forme de sels, il peut s'agir soit de sels de zinc, d'un métal alcalin ou alcalino-terreux, ou d'une amine organique lorsqu'ils comportent au moins une fonction acide libre, soit de sels d'un acide minéral ou organique, notamment de chlorhydrate, de bromhydrate ou de citrate lorsqu'ils comportent au moins une fonction amine.

Les composés selon l'invention peuvent se présenter sous forme tautomère lorsque R′ et R″, pris ensemble, forment un radical oxo et que $R_7$ représente le radical

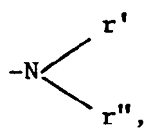

au moins l'un des substituants r′ et r″ étant un atome d'hydrogène, ces composés étant alors des lactames pouvant être représentés par la formule (III).

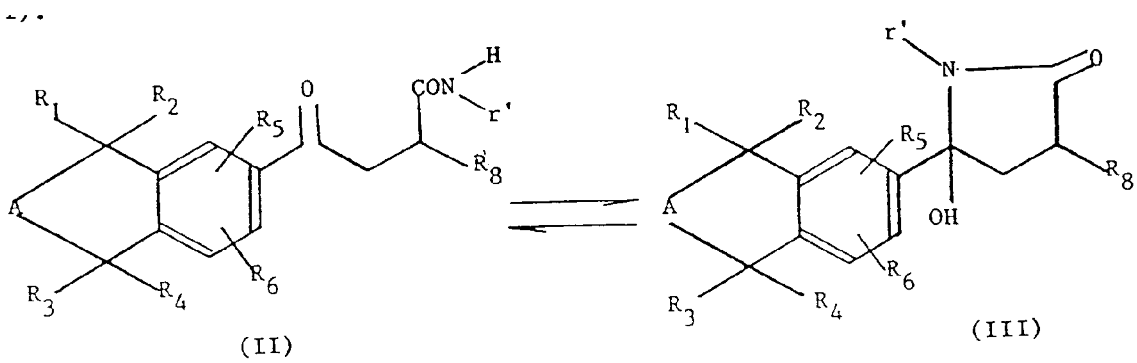

(II) (III)

Parmi les composés de formule (I) particulièrement préférés selon l'invention, on peut notamment mentionner ceux correspondant à la formule générale suivante :

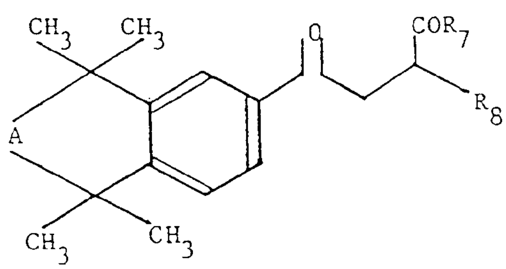

(IV)

dans laquelle :

A représente un radical $-(CH_2)_2-$ ou le radical

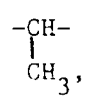

$R_7$ représente le radical $-OR_9$ ou

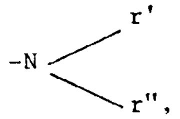

$R_9$ représentant un atome d'hydrogène ou un radical alkyle inférieur, r′ étant un atome d'hydrogène et r″ étant un radical alkyle ayant de 1 à 8 atomes de carbone, un radical monohydroxyalkyle, éventuellement interrompu par un hétéroatome ou un radical polyhydroxyalkyle ou r′ et r″, pris ensemble, forment avec l'atome d'azote un radical (hydroxy-2 éthyl)-4 pipérazinyle,
et $R_8$ représente un atome d'hydrogène ou un radical méthyle.

Parmi les composés de formule (I) selon l'invention, on peut notamment citer les suivants :

- L'acide (tétraméthyl-5,5,8,8 tétrahydro-5,6,7,8 naphtyl-2)-4 oxo-4 méthyl-2 butyrique,
- L'acide (méthano-5,8 tétrahydro-5,6,7,8 naphtyl-2)-4 oxo-4 méthyl-2 butyrique,
- L'acide (pentaméthyl-1,1,2,3,3 indanyl-5)-4 oxo-4 méthyl-2 butyrique,
- L'acide (tétraméthyl-1,1,3,3 indanyl-5)-4 oxo-4 méthyl-2 butyrique,
- L'acide (tétraméthyl-5,5,8,8 tétrahydro-5,6,7,8 naphtyl-2)-4 oxo-4 butyrique,
- L'acide (méthano-5,8 tétrahydro-5,6,7,8 naphtyl-2)-4 oxo-4 butyrique
- L'acide (pentaméthyl-1,1,2,3,3 indanyl-5)-4 oxo-4 butyrique,
- L'acide (tétraméthyl-1,1,3,3 indanyl-5)-4 oxo-4 butyrique,
- L'acide (diméthoxy-1,4 méthano-5,8 tétrahydro-5,6,7,8 naphtyl-2)-4 oxo-4 méthyl-2 butyrique,
- L'acide (diméthoxy-1,4 méthano-5,8 tétrahydro-5,6,7,8 naphtyl-2)-4 oxo-4 butyrique,
- Le (pentaméthyl-1,1,2,3,3 indanyl-5)-4 oxo-4 butyrate d'éthyle,
- Le (tétraméthyl-5,5,8,8 tétrahydro-5,6,7,8 naphtyl-2)-4 oxo-4 butyrate d'éthyle,
- Le (diméthoxy-1,4 méthano-5,8 tétrahydro-5,6,7,8 naphtyl-2)-4 oxo-4 butyrate d'éthyle,
- Le (tétraméthyl-5,5,8,8 tétrahydro-5,6,7,8 naphtyl-2)-4 oxo-4 butyrate d'éthyl-2′ hexyle,
- Le N-éthyl(tétraméthyl-5,5,8,8 tétrahydro-5,6,7,8 naphtyl-2)-4 oxo-4 méthyl-2 butyramide,
- Le N-éthyl(tétraméthyl-5,5,8,8 tétrahydro-5,6,7,8 naphtyl-2)-4 oxo-4 butyramide,
- Le N-éthyl (diméthoxy-1,4 méthano-5,8 tétrahydro-5,6,7,8 naphtyl-2)-4 oxo-4 butyramide,
- Le N-(hydroxy-2 éthyl)-4′ pipérazinyl(tétraméthyl-5,5,8,8 tétrahydro-5,6,7,8 naphtyl-2)-4 oxo-4 butyramide,
- Le N-(hydroxy-2 éthoxyéthyl)(tétraméthyl-5,5,8,8 tétrahydro-5,6,7,8 naphtyl-2)-4 oxo-4 butyramide,
- Le N-(hydroxy-2 éthoxyéthyl)(pentaméthyl-1,1,2,3,3 indanyl-5)-4 oxo-4 butyramide,
- L'acide (tétraméthyl-5,5,8,8 tétrahydro-5,6,7,8 naphtyl-2)-4 hydroxy-4 butyrique,
- L'acide (pentaméthyl,-1,1,2,3,3 indanyl-5)-4 hydroxy-4 butyrique,
- L'acide (diméthoxy-1,4 méthano-5,8 tétrahydro-5,6,7,8 naphtyl-2)-4 hydroxy-4 butyrique,
- Le (tétraméthyl-5,5,8,8 tétrahydro-5,6,7,8 naphtyl-2)-4 hydroxyimino-4 butyrate d'éthyle,
- L'acide (tétraméthyl-5,5,8,8 tétrahydro-5,6,7,8 naphtyl-2)-4 hydroxy-imino-4 butyrique,
- Le (tétraméthyl-5,5,8,8 tétrahydro-5,6,7,8 naphtyl-2)-4 oxo-4 butyrate de sodium,
- Le (tétraméthyl-5,5,8,8 tétrahydro-5,6,7,8 naphtyl-2)-4 oxo-4 méthyl-2 butyrate de sodium,
- Le (tétraméthyl-5,5,8,8 tétrahydro-5,6,7,8 naphtyl-2)oxo-4 butyrate de zinc,
- Le (diméthoxy-1,4 méthano-5,8 tétrahydro-5,6,7,8 naphtyl-2)-4 oxo-4 butyrate de zinc,
- Le (pentaméthyl-1,1,2,3,3 indanyl-5)oxo-4 butyrate de zinc,
- L'acide (tétraméthyl-5,5,8,8 tétrahydro-5,6,7,8 naphtyl-2)-4 butyrique,
- Le (tétraméthyl-5,5,8,8 tétrahydro-5,6,7,8 naphtyl-2)-4 oxo-4 méthyl-2 butyrate de zinc.

La présente invention a également pour objet le procédé de préparation des composés de formule (I).

Ces composés peuvent être préparés selon le schéma réactionnel suivant :

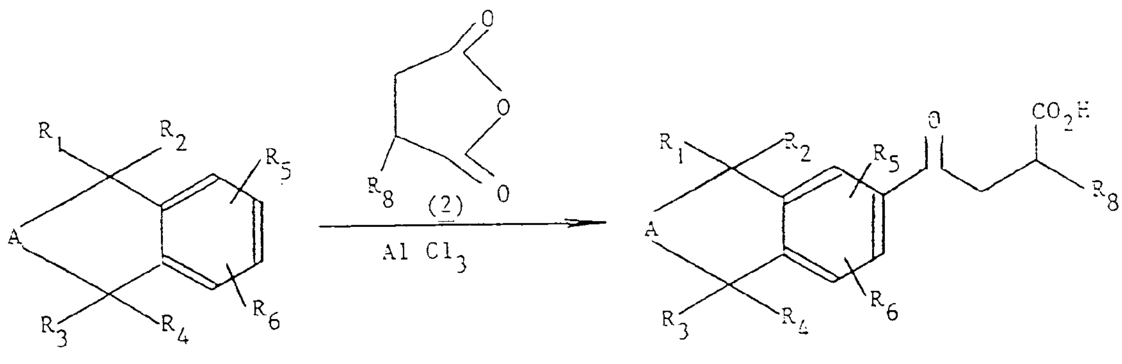


(1) (3)

Ce procédé consiste en une réaction de condensation dans les conditions de la réaction de Friedel Crafts d'un anhydride de structure (2) sur un composé aromatique bicyclique de formule (1).

Cette réaction de condensation est effectuée en présence d'un acide de Lewis tel que le chlorure d'aluminium ou le chlorure d'étain dans un solvent chloré tel que le dichloro-1,2 éthane ou le dichlorométhane.

Parmi les composés aromatiques bicycliques de départ de formule (1), on peut citer le tétraméthyl-1,1,4,4 tétrahydro-1,2,3,4 naphtalène (décrit dans J.A.C.S. 62, 36-44, (1940)), le méthano-1,4 tétrahydro-1,2,3,4 naphtalène ou benzonorbornène (décrit dans J.O.C., 32, 893-901, (1967)), le dihydro-5,8 méthano-1,4 tétrahydro-1,2,3,4 naphtalène (produit commercial), le tétraméthyl-1,1,3,3 indane et le pentaméthyl-1,1,2,3,3 indane (décrits dans le brevet français n°1.392.804).

A partir du céto-acide de formule (3), on peut accéder, selon le schéma réactionnel suivant, aux autres formes des composés selon l'invention:

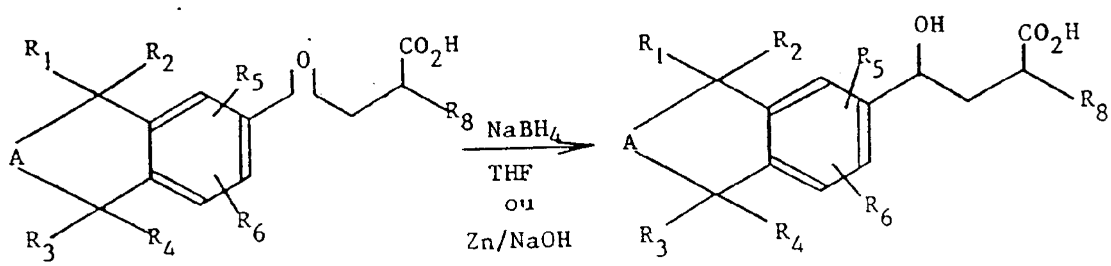


(3) (4)

(3)

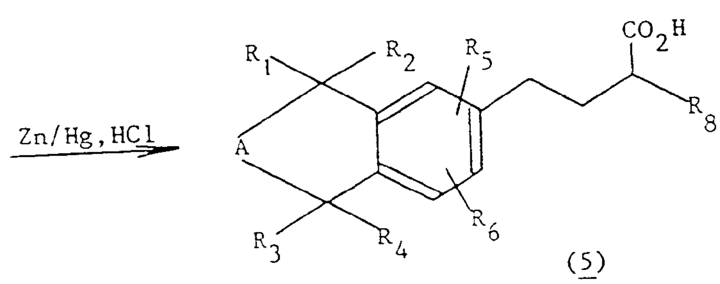


(5)

Ainsi, par réduction par le borohydrure de sodium dans un solvant tel que le tétrahydrofuranne ou bien par le zinc en milieu alcalin, on peut accéder aux alcools secondaires de formule (4).

Par réduction de Clemmensen, à l'aide d'amalgame de zinc en présence d'acide chlorhydrique, on peut accéder aux composés de formule (5).

Les dérivés acyloxy des composés de formule (I), (R′=acyloxy et R″=H), sont obtenus en faisant réagir une forme activée d'acide tel qu'un anhydride ou un chlorure d'acide sur un composé selon l'invention dans lequel R′=OH et R″=H.

Les dérivés alkoxy des composés de formule (I) (R′=alkoxy et R″=H), sont de même obtenus à partir des composés de formule (I) (R′=OH et R″=H), selon les méthodes connues.

Les composés de formule (I) dans laquelle R′ et R″ forment ensemble un radical hydroxyimino, sont obtenus par réaction du chlorhydrate d'hydroxylamine sur les composés carbonylés correspondants dans un solvant organique tel que l'éthanol, en présence d'une base minérale telle que le bicarbonate de sodium ou d'une base organique telle que la triéthylamine.

Les composés de formule (I), sous forme d'esters, d'amides ou de sels, sont préparés suivant les méthodes classiques d'estérification, d'amidification ou de salification.

Les composés de formule (I), selon la présente invention, présentent une excellente activité dans le test décrit par J. GIRARD et A. BARBIER, Int. Journal of Cosmetic Science, 2, 315-329 (1980) et M. GAUCI et J. OUSTRIN, Int. Journal of Cosmetic Science 3, 227-232 (1982). Ces auteurs ont en effet montré que le test "in vitro" d'incorporation de glucose marqué pouvait être retenu comme test d'orientation pour les anti-séborrhéiques non hormonaux puisque ce test rend compte de l'activité inhibitrice de la synthèse des lipides.

On sait par ailleurs qu'un accroissement de sécrétion du sébum produit des états dermatologiques tels que la séborrhée, les pellicules, la peau grasse, les cheveux gras, les points blancs et les points noirs.

Ces phénomènes chroniques des unités pilo-sébacées concernent surtout le visage, la poitrine et le dos.

En outre, les acides de formule (I) selon l'invention, dans laquelle ($R_7$ = OH), présentent une bonne activité sur "la souris Rhino", ce test étant généralement admis comme un des éléments du screening pour retenir un produit ayant une activité anti-acné.

Les composés selon l'invention conviennent donc tout particulièrement pour traiter les sécrétions excessives de sébum, notamment :

– les peaux et les cheveux gras,
– les acnés vulgaires, comédonniennes ou polymorphes,
– les acnés séniles, solaires et les acnés médicamenteuses ou professionnelles.

Les composés de formule (I) selon l'invention, trouvent donc une application dans le domaine cosmétique, en particulier l'hygiène corporelle et capillaire et, notamment, pour le traitement des peaux à tendance acnéique, pour la repousse des cheveux, l'anti-chute, pour lutter contre l'aspect gras de la peau ou des cheveux.

La présente invention a donc pour objet une composition cosmétique contenant, dans un support cosmétiquement acceptable, au moins un composé de formule (I), ou un de ses sels, ou un de ses isomères, cette composition se présentant notamment sous forme de lotion, gel, savon ou shampooing.

La concentration en composés de formule (I), dans les compositions cosmétiques est comprise entre 0,005 et 5% en poids et de préférence entre 0,01 et 1% en poids.

La présente invention a également pour objet une composition médicamenteuse, destinée notamment au traitement des différentes formes de l'acné, caractérisée par le fait qu'elle contient, dans un support pharmaceutiquement acceptable au moins un composé de formule (I) et/ou un de ses isomères et/ou un de ses sels.

Comme support des compositions, on peut utiliser tout support conventionnel, le composé actif se trouvant soit à l'état dissous soit à l'état dispersé dans le véhicule.

L'administration peut être effectuée par voie entérale, parentérale ou topique.

Par voie entérale, les médicaments peuvent se présenter sous forme de comprimés, de gélules, de dragées, de sirops, de suspensions, de solutions, de poudres, de granulés, d'émulsions.

Par voie parentérale, les compositions peuvent se présenter sous forme de solutions ou suspensions pour perfusions ou pour injections.

Les composés selon l'invention sont généralement administrés à une dose journalière d'environ 0,1mg/kg à 10mg/kg de poids corporel.

Par voie topique, les compositions pharmaceutiques à base des composés selon l'invention se présentent sous forme d'onguents, de teintures, de crèmes, de pommades, de poudres, de timbres, de tampons imbibés, de solutions, d'émulsions, de lotions, de gels, de sprays ou encore de suspensions.

Ces compositions par voie topique peuvent se présenter soit sous forme anhydre, soit sous forme aqueuse selon l'indication clinique.

Lorsque les composés selon l'invention sont utilisés par voie topique, on observe une bonne activité de ces composés sur une très grande gamme de dilution ; on peut utiliser notamment des concentrations en produits actifs allant de 0,01 à 10% en poids.

Il est bien entendu possible d'utiliser des concentrations supérieures lorsque ceci est rendu nécessaire pour une application thérapeutique particulière ; toutefois, les concentrations préférées en produits actifs sont comprises entre 0,1 et 5% en poids.

Les compositions cosmétiques et médicamenteuses selon l'invention, peuvent contenir des additifs inertes ou même cosmétiquement ou pharmacodynamiquement actifs et, notamment :

– des agents hydratants comme la thiamorpholinone et ses dérivés, ou l'urée,

– des agents anti-séborrhéiques tels que la S-carboxyméthylcystéine, la S-benzylcystéamine et leurs dérivés, la tioxolone,

– des agents anti-acnéiques comme le peroxyde de benzoyle,

– des agents antibiotiques comme l'érythromycine et ses esters, la néomycine, les tétracyclines ou les polyméthylène-4,5 isothiazolinones-3,

– des agents favorisant la repousse des cheveux comme le "Minoxidil" (diamino-2,4 pipéridino 6-pyrimidine oxyde-3) et ses dérivés, le Diazoxide (chloro-7 méthyl-3 benzothiadiazine-1,2,4 dioxide-1,1) et le Phénytoïn (diphényl-5,5 imidazolinedione-2,4) ou encore l'iodure d'oxapropanium;

– des agents anti-inflammatoires stéroïdiens et non stéroïdiens,

– des caroténoïdes et, notamment, le β-carotène,

– des agents anti-psoriasiques tels que l'anthraline et ses dérivés et les acides eicosatétraynoïque-5,8,11,14 et triynoïque-5,8,11 et leurs esters et amides.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des anti-oxydants tels que l'α-tocophérol, le butylhydroxy-anisole ou le butylhydroxy-toluène.

On va maintenant donner, à titre d'illustration et sans aucun caractère limitatif, plusieurs exemples de préparation des composés actifs de formule (I) selon l'invention ainsi que des exemples de compositions les contenant.

## EXEMPLE I

Préparation de l'acide (tétraméthyl-5,5,8,8 tétrahydro-5,6,7,8 naphtyl-2)-4 oxo-4 méthyl-2 butyrique

Formule (I) dans laquelle :

$$A= -(CH_2)_2- \quad ,$$

$R_1=R_2=R_3=R_4=-CH_3$ ; $R_5=R_5=H$ ; R',R"=oxo ; $R_7=OH$, $R_8=-CH_3$.

A une solution de 37,7g (0,2 mole) de tétraméthyl-1,1,4,4 tétrahydro-1,2,3,4 naphtalène et de 22,82g (0,2 mole) d'anhydride méthylsuccinique dans 250cm³ de dichloro-1,2 éthane anhydre, on ajoute par portions, en environ 1 heure, 53,3 g (0,4 mole) de chlorure d'aluminium anhydre de façon à maintenir la température inférieure ou égale à 30°C. Après 3 heures d'agitation à température ambiante, le milieu réactionnel est versé sur 200 cm³ d'eau glacée. La phase organique est décantée. La phase aqueuse est à nouveau extraite deux fois par 100 cm³ de dichlorométhane. Les phases de dichloroéthane et dichlorométhane sont rassemblées, lavées à l'eau, séchées sur sulfate de sodium et concentrées sous pression réduite. Le résidu est repris dans 300cm³ d'hexane, essoré, lavé trois fois successivement par 200cm³ de toluène puis recristallisé dans l'éther isopropylique. On obtient ainsi, après séchage, 20,7 g d'acide (tétraméthyl-5,5,8,8 tétrahydro-5,6,7,8 naphtyl-2)-4 oxo-4 méthyl-2 butyrique sous la forme d'un solide blanc dont le point de fusion est de 165°C.

Les spectres RMN$^1$H et $^{13}$C correspondent à la structure attendue.

Analyse élémentaire : $C_{19}H_{26}O_3$

| | C | H | O |
|---|---|---|---|
| Calculé | 75,46 | 8,67 | 15,87 |
| Trouvé | 75,22 | 8,72 | 16,00 |

## EXEMPLE II

Préparation de l'acide (méthano-5,8 tétrahydro-5,6,7,8 naphtyl-2)-4 oxo-4 méthyl-2 butyrique

Formule (I) dans laquelle :

$$A=-(CH_2)_2- ; R_1,R_3=-(CH_2)- ;$$

$R_2=R_4=H$ ; $R_5=R_6=H$ ; R',R"=oxo ; $R_7=-OH$, $R_5= -CH_3$

A une suspension de 13,33g (0,1 mole) de chlorure d'aluminium anhydre dans 70cm³ de dichlorométhane

sec, agitée à température ambiante, on ajoute goutte à goutte en environ 30mn une solution équimolaire de 7,11g (0,05 mole) de méthano-1,4 tétrahydro-1,2,3,4 naphtalène et de 5,7g (0,05 mole) d'anhydride méthylsuccinique dans 50cm$^3$ de dichlorométhane sec. L'addition terminée, on agite 1 heure supplémentaire à température ambiante puis verse dans 100cm$^3$ d'eau glacée. La phase organique est décantée et la phase aqueuse est à nouveau extraite deux fois par 100cm$^3$ de dichlorométhane. Les phases de dichlorométhane sont lavées à l'eau, séchées sur sulfate de sodium puis évaporées à sec. L'huile brute obtenue est cristallisée par trituration dans 100cm$^3$ d'hexane tiède. Après filtration, le solide beige obtenu est recristallisé dans 120cm$^3$ de mélange hexane/acétone (90/10). On isole ainsi 5,1g de cristaux blancs qui sont repris par 50cm$^3$ d'éther isopropylique, essorés et à nouveau recristallisés dans 80cm$^3$ de mélange éther isopropylique/hexane (60/40). Après séchage, on obtient 1,7g d'acide (méthano-5,8 tétrahydro-5,6,7,8 naphtyl-2)-4 oxo-4 méthyl-2 butyrique sous la forme d'un solide blanc dont le point de fusion est de 148°C.

Les spectres RMN$^1$H 250 MHz et $^{13}$C sont conformes à la structure attendue.
Analyse élémentaire : $C_{16}H_{18}O_2$

| | C | H | O |
|---|---|---|---|
| Calculé | 74,39 | 7,02 | 18,58 |
| Trouvé | 74,69 | 7,20 | 18,06 |

## EXEMPLE III

### Préparation de l'acide (tétraméthyl-5,5,8,8 tétrahydro-5,6,7,8 naphtyl-2)-4 oxo-4 butyrique

Formule (I) dans laquelle :

$A=-(CH_2)_2-$ ;

$R_1=R_2=R_3=R_4=-CH_3$ ; $R_5=R_5=H$ ; R′,R″=oxo ; $R_7=OH$ ; $R_8=H$

A une suspension de 13,18g (0,07mole) de tétraméthyl-1,1,4,4 tétrahydro-1,2,3,4 naphtalène et de 7g (0,07mole) d'anhydride succinique dans 150cm$^3$ de dichloro-1,2 éthane anhydre, on ajoute par portions, en environ 45mn, 18,7g (0,14mole) de chlorure d'aluminium anhydre de façon à maintenir la température inférieure à 30°C. Après 3 heures d'agitation, le milieu réactionnel est versé dans 100cm$^3$ d'eau glacée. La phase organique est décantée et la phase aqueuse est extraite trois fois par 100cm$^3$ de dichlorométhane. Les phases de dichloroéthane et dichlorométhane sont rassemblées, lavées à l'eau, séchées sur sulfate de sodium et concentrées sous pression réduite. Le solide brut obtenu est recristallisé deux fois dans l'acétone. On obtient ainsi 15,7g de cristaux blancs d'acide (tétraméthyl-5,5,8,8 tétrahydro-5,6,7,8 naphtyl-2)-4 oxo-4 butyrique dont le point de fusion est de 178°C.

Le spectre RMN$^1$H 250 MHz est conforme à la structure attendue.
Analyse élémentaire : $C_{18}H_{24}O_3$

| | C | H | O |
|---|---|---|---|
| Calculé | 74,97 | 8,39 | 16,45 |
| Trouvé | 74,76 | 8,51 | 16,30 |

## EXEMPLE IV

### Préparation de l'acide (pentaméthyl-1,1,2,3,3 indanyl-5)-4 oxo-4 butyrique

Formule (I) dans laquelle:

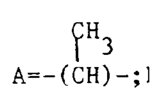

$R_1=R_2=R_4=-CH_3$ ; $R_5=R_5=H$ ; R′,R″=oxo ; $R_7=OH$ ; $R_8=H$

A une solution de 11g de pentaméthyl-1,1,2,3,3 indane (0,058mole) et de 7g d'anhydride succinique (0,07 mole) dans 100cm³ de dichloro-1,2 éthane anhydre, agitée à 0°C, on ajoute par petites portions 9,4g de chlorure d'aluminum de façon à ce que la température reste inférieure à 30°C. Le mélange réactionnel est ensuite agité pendant 3 heures à température ambiante puis versé dans 250cm³ d'eau glacée. La phase organique est décantée et la phase aqueuse est extraite deux fois au dichloro-1,2 éthane. Les phases organiques sont rassemblées, lavées à l'eau, séchées sur sulfate de magnésium. Le solvant est évaporé sous pression réduite et le produit obtenu est cristallisé dans l'éther isopropylique. On obtient 7g d'acide (pentaméthyl-1,1,2,3,3 indanyl-5)-4 oxo-4 butyrique sous forme de cristaux blancs dont le point de fusion est de 161°C.
Analyse élémentaire : $C_{18}H_{24}O_3$

| | C | H | O |
|---|---|---|---|
| Calculé | 74,97 | 8,39 | 16,64 |
| Trouvé | 74,92 | 8,50 | 16,49 |

EXEMPLE V

Préparation de l'acide (méthano-5,8 tétrahydro-5,6,7,8 naphtyl-2)-4 oxo-4 butyrique

Formule (I) dans laquelle

$$A : -(CH_2)_2- ; \quad R_1,R_3 = -(CH_2)- ;$$

$R_2$=$R_4$=H ; $R_5$=$R_6$=H ; R′, R″=oxo ; $R_7$=-OH ; $R_8$=H

A une solution agitée à 0°C de 30g de méthano-1,4 tétrahydro-1,2,3,4 naphtalène (0,2mole) et de 21g d'anhydride succinique (0,21mole) dans 300cm³ de dichlorométhane, on ajoute, par petites portions, en environ 2 heures, 56g de chlorure d'aluminium. L'agitation est ensuite maintenue 2 heures à température ambiante. Le milieu réactionnel est alors versé sur un volume égal d'eau glacée et la phase organique est décantée. La phase aqueuse est extraite deux fois par 300cm³ de dichlorométhane. Les phases de dichlorométhane sont rassemblées, lavées une fois à l'eau, décantées, séchées sur sulfate de magnésium et concentrées sous pression réduite. Le produit obtenu est déposé sur une colonne de gel de silice. L'acide attendu est entraîné à l'acétate d'éthyle, puis recristallisé dans l'hexane. On obtient 25g d'acide (méthano-5,8 tétrahydro-5,6,7,8 naphtyl-2)-4 oxo-4 butyrique sous forme de cristaux blancs de point de fusion : 135°C.
Analyse élémentaire : $C_{15}H_{16}O_3$

| | C | H | O |
|---|---|---|---|
| Calculé | 73,75 | 6,60 | 19,65 |
| Trouvé | 73,78 | 6,66 | 19,43 |

EXEMPLE VI

Préparation de l'acide (diméthoxy-1,4 méthano-5,8 tétrahydro-5,6,7,8 naphtyl-2)-4 oxo-4 butyrique

Formule (I) dans laquelle

$$A = -(CH_2)_2- \quad ; \quad R_1,R_3 = -(CH_2)- \quad ;$$

$R_2$=$R_4$=H, $R_5$=$R_5$=-$OCH_3$ ; R′,R″=oxo ; $R_7$=-OH ; $R_5$=H

A une solution agitée à 0°C de 30g de diméthoxy-1,4 méthano-5,8 tétrahydro-5,6,7,8 naphtalène (0,154mole) et de 17g d'anhydride succinique (0,17mole) dans 280cm³ de dichloro-1,2 éthane anhydre on ajoute en environ une heure par petites portions 23,5g de chlorure d'aluminium. A la fin de l'addition, le mélange est agité trois heures à température ambiante, puis versé sur 500cm³ d'eau glacée. La phase de dichloro-1,2 éthane est décantée et la phase aqueuse est extraite deux fois par 250cm³ de dichloro-1,2 éthane. Les phases organiques sont rassemblées, séchées sur sulfate de magnésium et concentrées. On obtient 30g de produit brut qui est recristallisé dans l'éther isopropylique. On isole ainsi 16g d'acide sulfate de magnésium et concentrées. On obtient 30g de produit brut qui est (diméthoxy-1,4 méthano-5,8 tétrahydro-5,6,7,8 naphtyl-2)-4 oxo-4 butyrique sous forme de cristaux blancs de point de fusion : 132°C.
Analyse élémentaire : $C_{17}H_{20}O_5$

| | C | H | O |
|---|---|---|---|
| Calculé | 67,09 | 6,62 | 26,29 |
| Trouvé | 66,74 | 6,53 | 25,88 |

EXEMPLE VII

Préparation du (pentaméthyl-1,1,2,3,3 indanyl-5)-4 oxo-4 butyrate d'éthyle

Formule (I) dans laquelle

$$A = -\overset{CH_3}{\overset{|}{(CH)}}- \ ;$$

$R_1$=$R_2$=$R_3$=$R_4$=-$CH_3$; $R_5$=$R_6$=H ; R',R''=oxo ; $R_7$=-$OC_2H_5$; $R_8$=H

Une solution de 2g (7mmoles) d'acide (pentaméthyl-1,1,2,3,3 indanyl-5)-4 oxo-4 butyrique décrit à l'exemple IV, dans 100cm³ d'alcool éthylique absolu contenant 0,2cm³ d'acide sulfurique à 98% est chauffée 4 heures au reflux. La solution est alors concentrée sous pression réduite. L'ester brut est dissous dans 100cm³ de dichlorométhane et la solution est lavée au bicarbonate de sodium puis à l'eau et séchée sur sulfate de sodium. Après évaporation à sec et après séchage, on obtient 1,9g de (pentaméthyl-1,1,2,3,3 indanyl-5)-4 oxo-4 butyrate d'éthyle sous la forme d'un liquide incolore.

Le spectre RMN¹H 80MHz est conforme à la structure attendue.
Analyse élémentaire : $C_{20}H_{28}O_3$

| | C | H | O |
|---|---|---|---|
| Calculé | 75,91 | 8,92 | 15,17 |
| Trouvé | 75,98 | 8,92 | 15,28 |

EXEMPLE VIII

Préparation du (tétraméthyl-5,5,8,8 tétrahydro-5,6,7,8 naphtyl-2)-4 oxo-4 butyrate d'éthyl-2' hexyle

Formule (I) dans laquelle

$$A = -(CH_2)_2- \ ;$$

$R_1$=$R_2$=$R_3$=$R_4$=-$CH_3$ ; $R_8$=$R_8$=H ; R',R''=oxo ; $R_7$=-$OC_8H_{17}$ ; $R_8$=H.

Une solution de 2,88g (10mmoles) d'acide (tétraméthyl-5,5,8,8 tétrahydro-5,6,7,8 naphtyl-2)-4 oxo-4 butyrique, décrit à l'exemple III, dans 120cm³ de toluène contenant 2g (20mmoles) d'éthyl-2 hexanol-1 et 0,1cm³ d'acide sulfurique à 98% est chauffée au reflux 4 heures avec distillation azéotropique de l'eau formée. La solution est alors refroidie, transférée dans une ampoule à décanter, lavée abondamment à l'eau, séchée sur sulfate de sodium et concentrée sous pression réduite. Le résidu est purifié rapidement par passage sur un filtre de gel de silice 60 en utilisant un mélange éluant dichlorométhane/toluène (60/40). Après évaporation sous vide et séchage prolongé, on obtient 3,6 g de (tétraméthyl-5,5,8,8 tétrahydro-5,6,7,8 naphtyl-2)-4 oxo-4 butyrate d'éthyl-2' hexyle sous la forme d'un liquide incolore.

Le spectre RMN¹H 80MHz correspond à la structure attendue.
Analyse élémentaire : $C_{26}H_{40}O_3$

| | C | H | O |
|---|---|---|---|
| Calculé | 77,95 | 10,07 | 11,98 |
| Trouvé | 78,13 | 9,97 | 11,79 |

EXEMPLE IX

Préparation du (tétraméthyl-5,5,8,8 tétrahydro-5,6,7,8 naphtyl-2)-4 oxo-4 butyrate d'éthyle

Formule (I) dans laquelle

$$A=-(CH_2)_2- ; I$$

$R_1=R_2=R_3=R_4=-CH_3$ ; $R_5=R_5=H$ ; R',R''=oxo ; $R_7=-OC_2H_5$ ; $R_8=H$

Une solution de 1,01g (3,5mmoles) d'acide (tétraméthyl-5,5,8,8 tétrahydro-5,6,7,8 naphtyl-2)-4 oxo-4 butyrique, décrit à l'exemple III, dans 40cm³ d'alcool éthylique contenant 0,06 cm³ d'acide sulfurique à 98% est chauffée 5 heures au reflux puis concentrée sous pression réduite. L'ester brut est dissous dans 50cm³ de dichlorométhane. La solution est lavée au bicarbonate de sodium puis à l'eau, séchée sur sulfate de sodium et évaporée à sec. Après séchage, on obtient 0,9g de (tétraméthyl-5,5,8,8 tétrahydro-5,6,7,8 naphtyl-2)-4 oxo-4 butyrate d'éthyle sous la forme d'un liquide épais incolore.

Le spectre RMN$^1$H 80MHz est conforme à la structure attendue.

Analyse élémentaire : $C_{20}H_{28}O_3$

| | C | H | O |
|---|---|---|---|
| Calculé | 75,91 | 8,92 | 15,17 |
| Trouvé | 75,88 | 8,87 | 15,23 |

EXEMPLE X

Préparation du N-éthyl(tétraméthyl-5,5,8,8 tétrahydro-5,6,7,8 naphtyl-2)-4 oxo-4 méthyl-2 butyramide

Formule (I) dans laquelle

$$A=-(CH_2)_2- ;$$

$R_1=R_2=R_3=R_4=-CH_3$ ; $R_5=R_5=H$ ; R', R''=oxo ; $R_7$=-NHEt ; $R_8=-CH_3$

A une suspension de 3g (10mmoles) d'acide (tétraméthyl-5,5,8,8 tétrahydro-5,6,7,8 naphtyl-2)-4 oxo-4 méthyl-2 butyrique dans 60cm³ de dichlorométhane anhydre, on ajoute 1,95g (12mmoles) de N,N'-carbonyl diimidazole et agite 1h30 à température ambiante. La solution obtenue est refroidie à +5°C. On ajoute alors 0,86cm³ (13mmoles) d'éthylamine anhydre et maintient l'agitation 3 heures en laissant revenir à température ambiante. Le milieu réactionnel est transféré dans une ampoule à décanter et lavé à l'acide chlorhydrique 0,1N puis à l'eau. La phase de dichlorométhane est séchée sur sulfate de sodium et concentrée sous pression réduite. L'huile brute obtenue est purifiée par chromatographie sur gel de silice 60 dans un mélange toluène/dichlorométhane/acétate d'éthyle (5/3/2). Après évaporation et séchage, on isole 0,6g de N-éthyl(tétraméthyl-5,5,8,8 tétrahydro-5,6,7,8 naphtyl-2)-4 oxo-4 méthyl-2 butyramide sous la forme d'un solide blanc dont de point de fusion est de 101°C.

Les spectres I.R. et RMN$^1$H 250MHz correspondent à la structure attendue.

Analyse élémentaire : $C_{21}H_{31}NO_2$

| | C | H | N | O |
|---|---|---|---|---|
| Calculé | 76,55 | 9,48 | 4,25 | 9,71 |
| Trouvé | 76,53 | 9,54 | 4,16 | 9,84 |

EXEMPLE XI

Préparation du N-(hydroxy-2 éthyl)-4′ pipérazinyl (tétraméthyl-5,5,8,8 tétrahydro-5,6,7,8 naphtyl-2)-4 oxo-4 butyramide

Formule (I) dans laquelle $A=-(CH_2)_2-$ ; $R_1=R_2=R_3=R_4=-CH_3$ ; $R_5=R_6=H$ ; R',R''=oxo ; $R_7$ = 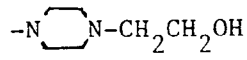 ; $R_8=H$

Une suspension de 2,88g (10mmoles) d'acide (tétraméthyl-5,5,8,8 tétrahydro-5,6,7,8 naphtyl-2)-4 oxo-4 butyrique et de 1,95g (12mmoles) de N,N′-carbonyl diimidazole dans 50cm³ de dichlorométhane anhydre est agitée 1h30 à température ambiante. La solution obtenue est alors refroidie à +5°C et 1,47cm³ (12mmoles) de N-hydroxy-2 éthylpipérazine sont ajoutés. L'agitation est maintenue 2 heures en laissant revenir à température ambiante. Le milieu réactionnel est transféré dans une ampoule à décanter et lavé abondamment à l'eau. La phase de dichlorométhane est séchée sur sulfate de sodium et concentrée sous pression réduite. Le solide brut obtenu est purifié par chromatographie sur gel de silice 60 dans le tétrahydrofuranne. Après évaporation et séchage, on isole 1,7g de N-(hydroxy-2 éthyl)-4′ pipérazinyl (tétraméthyl-5,5,8,8 tétrahydro-5,6,7,8 naphtyl-2)-4 oxo-4 butyramide sous la forme d'une poudre blanche dont le point de fusion est de 115°C.

Le spectre RMN¹H 250MHz est conforme à la structure attendue.

Analyse élémentaire : $C_{22}H_{36}N_2O_3$

| | C | H | N | O |
|---|---|---|---|---|
| Calculé | 71,96 | 9,06 | 7,00 | 11,98 |
| Trouvé | 72,01 | 9,11 | 7,04 | 12,03 |

EXEMPLE XII

Préparation du N-(hydroxy-2 éthoxyéthyl) (tétraméthyl-5,5,8,8 tétrahydro-5,6,7,8 naphtyl-2)-4 oxo-4 butyramide

Formule (I) dans laquelle

$$A=-(CH_2)_2- ;$$

$R_1=R_2=R_3=R_4=-CH_3$ ; $R_5=R_5=H$ ;R′,R″=oxo ;$R_7=-NH-(CH_2)_2-O-(CH_2)_2-OH$ ; $R_8=H$

Une suspension de 2,88g (10mmoles) d'acide (tétraméthyl-5,5,8,8 tétrahydro-5,6,7,8 naphtyl-2)-4 oxo-4 butyrique, décrit à l'exemple III, et de 1,95g (12mmoles) de N,N′-carbonyldiimidazole dans 50cm³ de dichlorométhane anhydre est agitée 2 heures à température ambiante. La solution obtenue est refroidie entre +5 et +10°C et 1,26g (12mmoles) de N-hydroxy-2 éthoxyéthylamine sont ajoutés. L'agitation est maintenue 3 heures en laissant revenir à température ambiante. Le milieu réactionnel est transféré dans une ampoule à décanter et dilué à 100cm³. Après lavage à l'acide chorhydrique 0,1N puis à l'eau, la phase de dichlorométhane est séchée sur sulfate de sodium puis évaporée à sec. Le résidu est purifié par chromatographie sur gel de silice 60 dans un mélange éluant dichlorométhane/toluène/acétate d'éthyle/isopropanol (40/25/25/10). Après évaporation et séchage, on obtient 2,5g de N-(hydroxy-2 éthoxyéthyl) (tétraméthyl-5,5,8,8 tétrahydro-5,6,7,8 naphtyl-2)-4 oxo-4 butyramide sous la forme d'une huile épaisse incolore conservée à l'abri de la lumière.

Le spectre RMN¹H 250MHz est conforme à la structure attendue.

Analyse élémentaire : $C_{22}H_{33}NO_4$ ,O,5$H_2O$

| | C | H | N | O |
|---|---|---|---|---|
| Calculé | 68,72 | 8,91 | 3,64 | 18,72 |
| Trouvé | 68,70 | 8,95 | 3,65 | 18,66 |

EXEMPLE XIII

préparation de l'acide (tétraméthyl-5,5,8,8 tétrahydro-5,6,7,8 naphtyl-2)-4 hydroxy-4 butyrique

Formule (I) dans laquelle :

$$A = -(CH_2)_2- ;$$

$R_1=R_2=R_3=R_4=-CH_3$ ; $R_5=R_5=H$ ; $R'=OH$, $R''=H$, $R_7=-OH$ ; $R_8=H$

A une solution de 2,02g (7mmoles) d'acide (tétraméthyl-5,5,8,8 tétrahydro-5,6,7,8 naphtyl-2)-4 oxo-4 butyrique, décrit à l'exemple III, dans 60cm³ de tétrahydrofuranne anhydre, on ajoute 1,06g (21mmoles) de borohydrure de sodium et agite une nuit à température ambiante. Le milieu réactionnel est alors refroidi à environ 10°C et acidifié par addition lente d'acide chlorhydrique 0,1N. Le mélange est extrait à l'éther éthylique (3x100cm³). La phase organique est lavée à l'eau, séchée sur sulfate de sodium et évaporée à sec. Le solide obtenu est repris par 100cm³ d'hexane et essoré. On obtient ainsi 1,75g de produit attendu en mélange avec sa lactone. On ajoute 20cm³ de soude 0,5N et chauffe 1 heure au reflux. La solution est ensuite refroidie à température ambiante et acidifiée par addition de 0,6cm³ d'acide acétique glacial. Le précipité est essoré, lavé abondamment à l'eau et séché sous vide à 50°C. On obtient 1,7g d'acide (tétraméthyl-5,5,8,8 tétrahydro-5,6,7,8 naphtyl-2)-4 hydroxy-4 butyrique sous la forme d'un solide blanc dont le point de fusion est de 110°C.

Les spectres I.R. et RMN$^1$H 80MHz sont conformes à la structure attendue.

Analyse élémentaire : $C_{18}H_{26}O_3$

| | C | H | O |
|---|---|---|---|
| Calculé | 74,44 | 9,02 | 16,53 |
| Trouvé | 74,85 | 8,78 | 16,42 |

EXEMPLE XIV

Préparation du (tétraméthyl-5,5,8,8 tétrahydro-5,6,7,8 naphtyl-2)-4 hydroxyimino-4 butyrate d'éthyle

Formule (I) dans laquelle :

$$A = -(CH_2)_2- ;$$

$R_1=R_2=R_3=R_4=-CH_3$; $R_5=R_6=H$ ; $R',R''= =N-OH$ ; $R_7=-OC_2H_5$ ; $R_5=H$

A une solution de 9,96g (0,0315 mole) de (tétraméthyl-5,5,8,8 tétrahydro-5,6,7,8 naphtyl-2)-4 oxo-4 butyrate d'éthyle, décrit à l'exemple IX, dans 250cm³ d'alcool éthylique, on ajoute 4,38g (0,063 mole) de chlorhydrate d'hydroxylamine puis 8,9cm³ (0,063mole) de triéthylamine et chauffe 12 heures au reflux. Après concentration sous pression réduite, le résidu est repris par 200cm³ de dichlorométhane. La solution est lavée à l'acide chlorhydrique dilué puis à l'eau. La phase de dichlorométhane est séchée sur sulfate de sodium et évaporée à sec. Le produit brut est alors purifié par chromatographie sur gel de silice 60 dans un mélange toluène/dichlorométhane/tétrahydrofuranne (6/3/1). Après évaporation et séchage, on isole 6,6g de (tétraméthyl-5,5,8,8 tétrahydro-5,6,7,8 naphtyl-2)-4 hydroxyimino-4 butyrate d'éthyle sous la forme d'un liquide incolore.

Le spectre RMN$^1$H 80MHz est conforme à la structure attendue.

Analyse élémentaire : $C_{20}H_{29}NO_3$

| | C | H | N | O |
|---|---|---|---|---|
| Calculé | 72,47 | 8,82 | 4,23 | 14,48 |
| Trouvé | 72,46 | 8,90 | 4,15 | 14,43 |

EXEMPLE XV

Préparation de l'acide (tétraméthyl-5,5,8,8 tétrahydro-5,6,7,8 naphtyl-2)-4 hydroxyimino-4 butyrique

Formule (I) dans laquelle :

$A=-(CH_2)_2-$;

$R_1=R_2=R_3=R_4=-CH_3$; $R_5=R_6=H$ ; R′,R″= =N-OH ; $R_7=OH$ ; $R_8=H$

Une solution de 2g (6mmoles) de (tétraméthyl-5,5,8,8 tétrahydro-5,6,7,8 naphtyl-2)-4 hydroxyimino-4 butyrate d'éthyle, décrit à l'exemple XIV, dans un mélange de 30cm$^3$ d'alcool éthylique et 30cm$^3$ de potasse 2N est chauffée 2 heures à 60°C. Après refroidissement à température ambiante, on ajoute 100cm$^3$ d'eau et évapore l'alcool sous pression réduite. L'acide est précipité par addition d'acide chlorhydrique 12N. Après filtration, lavage à l'eau et séchage, le produit brut est recristallisé dans l'éther isopropylique. On obtient ainsi 1,4g de cristaux blancs d'acide (tétraméthyl-5,5,8,8 tétrahydro-5,6,7,8 naphtyl-2)-4 hydroxyimino-4 butyrique dont le point de fusion est de 146°C.

Le spectre RMN$^1$H 250MHz est conforme à la structure attendue.

Analyse élémentaire : $C_{18}H_{25}NO_3$

| | C | H | N | O |
|---|---|---|---|---|
| Calculé | 71,26 | 8,31 | 4,62 | 15,82 |
| Trouvé | 71,37 | 8,29 | 4,62 | 16,04 |

EXEMPLE XVI

Préparation de l'acide (tétraméthyl-5,5,8,8 tétrahydro-5,6,7,8 naphtyl-2)-4 butyrique

Formule (I) dans laquelle :

$A=-(CH_2)_2-$;

$R_1=R_2=R_3=R_4=-CH_3$; $R_5=R_6=H$ ; R′=R″=H ; $R_7=OH$ ; $R_8=H$

A un mélange de 6g de laine de zincet de 0,6g de chlorure mercurique, on ajoute 9cm$^3$ d'eau et 0,3cm$^3$ d'acide chlorhydrique 12N et agite 10mn à température ambiante. La phase aqueuse est éliminée par décantation. L'amalgame obtenu est rincé par 20cm$^3$ d'eau. On ajoute alors 7,21g (0,025mole) d'acide (tétraméthyl-5,5,8,8 tétrahydro-5,6,7,8 naphtyl-2)-4 oxo-4 butyrique décrit à l'exemple III, 10cm$^3$ d'eau, 8cm$^3$ de toluène, 5cm$^3$ d'acide chlorhydrique 12N et chauffe 34 heures au reflux sous agitation en ajoutant 3cm$^3$ d'acide chlorhydrique 12N toutes les 6 heures. Le milieu réactionnel est dilué par addition de 100cm$^3$ de toluène et filtré (séparation de l'amalgame résiduel) avec rinçage au toluène. La phase toluénique est séparée, lavée abondamment à l'eau, séchée sur sulfate de sodium et concentrée sous pression réduite. L'huile brute obtenue est purifiée par chromatographie sur gel de silice 60 dans le mélange éluant acide acétique/dioxanne/toluène 2/8/90. Après évaporation, le solide isolé est repris par le minimum d'éther isopropylique, essoré et séché sous vide à 70°C. On obtient ainsi 4,6g d'acide (tétraméthyl-5,5,8,8 tétrahydro-5,6,7,8 naphtyl-2)-4 butyrique sous la forme d'un solide blanc dont le point de fusion est de : 99°C.

Le spectre RMN$^1$H 80MHz est conforme à la structure attendue.

Analyse élémentaire : $C_{18}H_{26}O_2$

| | C | H | O |
|---|---|---|---|
| Calculé | 78,79 | 9,55 | 11,66 |
| Trouvé | 78,73 | 9,53 | 11,44 |

EXEMPLE XVII

Préparation du (tétraméthyl-5,5,8,8 tétrahydro-5,6,7,8 naphtyl-2-4 oxo-4 méthyl-2 butyrate de sodium

Formule (I) dans laquelle :

$A=-(CH_2)_2-$;

$R_1=R_2=R_3=R_4=-CH_3$; $R_5=R_6=H$ ; R′=R″=oxo ; $R_7=-O^{\ominus}Na^{\oplus}$ ; $R_8=-CH_3$

On met en suspension 1,028g (3,39mmoles) d'acide (tétraméthyl-5,5,8,8 tétrahydro-5,6,7,8 naphtyl-2)-4 oxo-4 méthyl-2 butyrique, décrit à l'exemple I, dans 350cm³ d'eau bipermutée, ajoute 33,9cm³ de soude aqueuse 0,1N (3,39mmoles) et agite en tiédissant jusqu'à dissolution. La solution obtenue est filtrée puis évaporée à sec sous pression réduite. On obtient ainsi, après séchage sous vide à 80°C, 1,09g de (tétraméthyl-5,5,8,8 tétrahydro-5,6,7,8 naphtyl-2)-4 oxo-4 méthyl-2 butyrate de sodium sous la forme d'un solide blanc devenant vitreux vers 80°C.

EXEMPLE XVIII

Préparation du (tétraméthyl-5,5,8,8 tétrahydro-5,6,7,8 naphtyl-2)-4 oxo-4 butyrate de sodium

Formule (I) dans laquelle :

$A=-(CH_2)_2-$ ; F

$R_1=R_2=R_3=R_4=-CH_3$; $R_5=R_6=H$ ; R',R"=oxo ; $R_7=-O^{\ominus}Na^{\oplus}$ ; $R_8=H$

On met en suspension 0,502g (1,74mmoles) d'acide (tétraméthyl-5,5,8,8 tétrahydro-5,6,7,8 naphtyl-2)-4 oxo-4 butyrique, décrit à l'exemple III, dans 200cm³ d'eau bipermutée, ajoute 17,4cm³ (1,74mmoles) de soude aqueuse 0,1N et agite en tiédissant jusqu'à dissolution. La solution obtenue est filtrée puis évaporée à sec. Après séchage sous vide à 80°C, on obtient 0,52g de (tétraméthyl-5,5,8,8 tétrahydro-5,6,7,8 naphtyl-2)-4 oxo-4 butyrate de sodium sous la forme d'un solide blanc dont le point de fusion est supérieur à 250°C.

EXEMPLE XIX

Préparation du (tétraméthyl-5,5,8,8 tétrahydro-5,6,7,8 naphtyl-2)-4 oxo-4 méthyl-2 butyrate de zinc

Formule (I) dans laquelle :

$A=-(CH_2)_2-$ ; I

$R_1=R_2=R_3=R_4=-CH_3$; $R_5=R_6=H$ ; R',R"=oxo ; $R_7=-O^{\ominus}$,1/2 $Zn^{2\oplus}$ ; $R_8=-CH_3$

On dissout 308mg (0,95mmole) de (tétraméthyl-5,5,8,8 tétrahydro-5,6,7,8 naphtyl-2)-4 oxo-4 méthyl-2 butyrate de sodium, obtenu à l'exemple XVII, dans 150cm³ d'eau puis ajoute 136,5mg (0,475mmole) de sulfate de zinc, $7H_2O$. Le sel de zinc précipite. Il est essoré, lavé à l'eau et séché sous vide à 70-80°C. On obtient ainsi 0,32g de (tétraméthyl-5,5,8,8 tétrahydro-5,6,7,8 naphtyl-2)-4 oxo-4 méthyl-2 butyrate de zinc sous la forme d'un solide blanc devenant vitreux vers 115°C.

EXEMPLE XX

Préparation du (tétraméthyl-5,5,8,8 tétrahydro-5,6,7,8 naphtyl-2)-4 oxo-4 butyrate de zinc

Formule (I) dans laquelle :

$A=-(CH_2)_2-$ ; F

$R_1=R_2=R_3=R_4=-CH_3$; $R_5=R_6=H$ ; R',R"=oxo ; $R_7=-O^{\ominus}$,1/2 $Zn^{2\oplus}$ ; $R_8=H$

On dissout 317mg (1,02 mmoles) de (tétraméthyl-5,5,8,8 tétrahydro-5,6,7,8 naphtyl-2)-4 oxo-4 butyrate de sodium, obtenu à l'exemple XVIII, dans 150cm³ d'eau puis ajoute 147mg (0,51mmole) de sulfate de zinc, $7H_2O$. Le précipité est essoré, lavé à l'eau et séché sous vide à 80°C. On obtient ainsi 0,33g de (tétraméthyl-5,5,8,8 tétrahydro-5,6,7,8 naphtyl-2)-4 oxo-4 butyrate de zinc sous la forme d'un solide blanc devenant vitreux vers 125°C.

EXEMPLES DE FORMULATION

1 - Lotion anti-séborrhéique

Alcool absolu....................................59,0g
Propylène glycol.................................40,0g
Acide (tétraméthyl-5,5,8,8 tétrahydro-5,6,7,8 naphtyl-2)-4 oxo-4 butyrique........................ 1,0g

2 - Lotion contre les peaux grasses

Alcool absolu....................................60,0g
Polyéthylène glycol.............................. 39,5g
Acide (tétraméthyl-5,5,8,8 tétrahydro-5,6,7,8 naphtyl-2)-4 oxo-4 méthyl-2 butyrique................ 0,5g

3 - Gel pour lutter contre la peau grasse à tendance acnéique

Carbopol 941...................................... 0,8g
Alcool absolu..................................... 32,15g
Propylène glycol.................................. 35,0g
Butylhydroxytoluène............................... 0,02g
Butylhydroxyanisole............................... 0,03g
Triéthanolamine 20%............................... 1,0g
Eau purifiée...................................... 30,0g
Acide (tétraméthyl-5,5,8,8 tétrahydro-5,6,7,8 naphtyl-2)-4 oxo-4 butyrique........................ 1,0g

4 - Gel pour lutter contre les peaux grasses à tendance acnéique

Klucel H (dérivé cellulosique)..................... 1,0g
Alcool absolu..................................... 69,0g
Propylène glycol.................................. 28,45g
Butylhydroxytoluène............................... 0,02g
Butylhydroxyanisole............................... 0,03g
N-(hydroxy-2 éthoxyéthyl) (tétraméthyl-5,5,8,8 tétrahydro-5,6,7,8 naphtyl-2)-4 oxo-4 butyramide..... 1,5g

5 - Crème pour peaux grasses

| | |
|---|---|
| Monostéarate de glycol | 4,0g |
| Alcool cétylique | 3,5g |
| Myrj 53 stéarate de polyéthylène glycol (50moles d'OE) vendu par la Société ATLAS | 3,0g |
| Triglycérides caprique/caprylique | 22,0g |
| Parahydroxybenzoate de propyle | 0,15g |
| Butylhydroxytoluène | 0,02g |
| Butylhydroxyanisole | 0,03g |
| Propylène glycol | 8,0g |
| (Tétraméthyl-5,5,8,8 tétrahydro-5,6,7,8 naphtyl-2)-4 oxo-4 butyrate d'éthyl-2' hexyle | 2,0g |
| Eau q.s.p | 100,0g |

6 - Stick (teinté) pour peaux grasses

| | |
|---|---|
| Vaseline | 19,4g |
| Cosbiol (perhydrosqualène) | 40,0g |
| Paraffine solide | 2,0g |
| Cire de Carnauba | 2,0g |
| Ozokérite | 9,0g |
| Butylhydroxyanisole | 0,05g |
| Butylhydroxytoluène | 0,05g |
| Oxyde de fer rouge | 0,5g |
| Oxyde de fer jaune | 1,5g |
| Oxyde de fer brun | 2,5g |
| Oxyde de titane | 20,0g |
| Acide (tétraméthyl-5,5,8,8 tétrahydro-5,6,7,8 naphtyl-2)-4 hydroxy-4 butyrique | 1,0g |
| Amidon de riz | 2,0g |

## Revendications

1. Dérivés aromatiques d'acide butyrique caractérisés par le fait qu'ils répondent à la formule suivante :

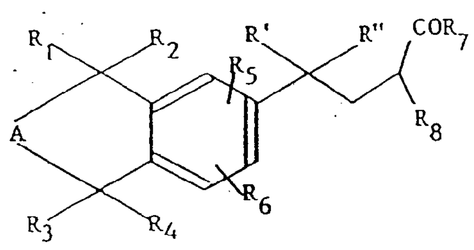

(I)

dans laquelle :

$R_1$, $R_2$, $R_3$ et $R_4$ représentent, indépendamment l'un de l'autre, un atome d'hydrogène ou un radical alkyle inférieur, au moins deux des radicaux $R_1$ à $R_4$ étant différents d'un atome d'hydrogène,

A représente un radical méthylène ou diméthylène, substitué ou non par un radical alkyle inférieur ; lorsque A représente un radical diméthylène, $R_1$ et $R_3$ peuvent former ensemble un radical méthylène ou diméthylène,

$R_5$ et $R_6$ représentent un atome d'hydrogène, un atome d'halogène, un radical alkyle inférieur, un radical alkoxy inférieur ou un radical hydroxyle;

R′ représente un atome d'hydrogène, un radical hydroxyle, un radical alkoxy inférieur ou un radical acyloxy en $C_1$-$C_4$,

R″ représente un atome d'hydrogène ou un radical alkoxy inférieur, ou R′ et R″, pris ensemble, forment un radical oxo (=O) ou hydroxyimino (=N-OH),

$R_8$ représente un atome d'hydrogène ou un radical alkyle inférieur, et $R_7$ représente le radical -$OR_9$ ou

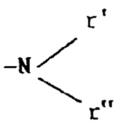

,

$R_9$ représentant un atome d'hydrogène, un radical alkyle ayant de 1 à 20 atomes de carbone, un radical monohydroxyalkyle, polyhydroxyalkyle, aryle ou aralkyle, un reste de sucre, un radical carboxyalkyle ou alkoxycarbonylalkyle,

r′ et r″ représentant un atome d'hydrogène, un radical alkyle inférieur, un radical monohydroxyalkyle éventuellement interrompu par un hétéroatome, un radical polyhydroxyalkyle, un radical aryle ou benzyle, un reste d'amino-acide ou de sucre aminé ou, pris ensemble, forment un hétérocycle éventuellement substitué par un radical hydroxyalkyle en $C_1$-$C_3$, et les sels desdits composés de formule (I) et leurs isomères optiques ainsi que les formes tautomères des composés de formule (I).

2. Composés selon la revendication 1 caractérisés par le fait que les radicaux alkyles inférieurs et ceux ayant jusqu'à 20 atomes de carbone sont pris dans le groupe constitué par les radicaux méthyle, éthyle, propyle, isopropyle, butyle, tertiobutyle, éthyl-2 hexyle, octyle, dodécyle, hexadécyle et octadécyle.

3. Composés selon la revendication 1 caractérisés par le fait que le radical monohydroxyalkyle est le radical hydroxy-2 éthyle, hydroxy-2 propyle ou hydroxy-2 éthoxyéthyle.

4. Composés selon la revendication 1 caractérisés par le fait que le radical polyhydroxyalkyle est le radical dihydroxy-2,3 propyle, dihydroxy-1,3 propyl-2 ou le reste du pentaérythritol.

5. Composés selon la revendication 1 caractérisés par le fait que le radical alkoxy inférieur est le radical méthoxy, isopropoxy, butoxy ou tert-butoxy.

6. Composés selon la revendication 1 caractérisés par le fait que le reste d'un sucre dérive du glucose, du mannose, de l'érythrose ou du galactose.

7. Composés selon la revendication 1 caractérisés par le fait que le reste de sucre aminé dérive de glucosamine, de galactosamine, de mannosamine ou de méglumine.

8. Composés selon la revendication 1 caractérisés par le fait que les radicaux r′ et r″, pris ensemble, forment avec l'atome d'azote auquel ils sont reliés un radical pipéridino, pipérazino, morpholino, pyrrolidino ou (hydroxy-2 éthyl)-4 pipérazino.

9. Composés selon l'une quelconque des revendications caractérisés par le fait qu'ils répondent à la formule suivante :

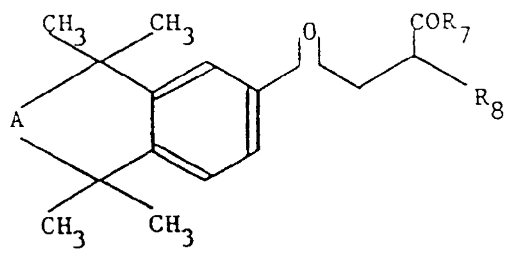

(IV)

dans laquelle :

A représente un radical

$-(CH_2)_2-$

ou le radical

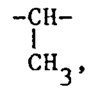

$R_7$ représente le radical $-OR_9$ ou

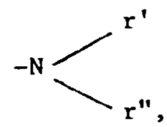

$R_9$ représentant un atome d'hydrogène ou un radical alkyle inférieur, r' étant un atome d'hydrogène et r" étant un radical alkyle ayant de 1 à 8 atomes de carbone, un radical monohydroxyalkyle, éventuellement interrompu par un hétéroatome ou un radical polyhydroxyalkyle ou r' et r", pris ensemble, forment avec l'atome d'azote un radical (hydroxy-2 éthyl)-4 pipérazinyle,

et $R_8$ représente un atome d'hydrogène ou un radical méthyle.

10. Composés selon l'une quelconque des revendications 1 à 9, caractérisés par le fait qu'ils sont pris dans le groupe constitué par :
l'acide (tétraméthyl-5,5,8,8-tétrahydro-5,6,7,8 naphtyl-2)-4 oxo-4 méthyl-2 butyrique,
l'acide (tétraméthyl-5,5,8,8-tétrahydro-5,6,7,8 naphtyl-2)-4 oxo-4 butyrique,
le (tétraméthyl-5,5,8,8-tétrahydro-5,6,7,8-naphtyl-2)-4 oxo-4 butyrate d'éthyle,
le (tétraméthyl-5,5,8,8-tétrahydro-5,6,7,8-naphtyl-2)-4 oxo-4 butyrate d'éthyl-2' hexyle,
le N-éthyl(tétraméthyl-5,5,8,8-tétrahydro-5,6,7,8 naphtyl-2)-4 oxo-4 méthyl-2 butyramide,
le N-éthyl(tétraméthyll-5,5,8,8-tétrahydro-5,6,7,8 naphtyl-2)-4 oxo-4 butyramide,
le N-(hydroxy-2 éthyl)-4'pipérazinyl(tétraméthyl-5,5,8,8-tétrahydro-5,6,7,8 napthyl-2)-4 oxo-4 butyramide,
le N-(hydroxy-2 éthoxyéthyl)(tétraméthyl-5,5,8,8-tétrahydro-5,6,7,8 naphtyl-2)-4 oxo-4 butyramide,
l'acide (tétraméthyl-5,5,8,8-tétrahydro-5,6,7,8 naphtyl-2)-4 hydroxy-4 butyrigue,
le (tétraméthyl-5,5,8,8-tétrahydro-5,6,7,8 naphtyl-2)-4 hydroxyimino-4 butyrate d'éthyle,
l'acide (tétraméthyl-5,5,8,8-tétrahydro-5,6,7,8 naphtyl-2)-4 hydroxyimino-4 butyrique,
le (tétraméthyl-5,5,8,8-tétrahydro-5,6,7,8 naphtyl-2)-4 oxo-4 butyrate de sodium,
le (tétraméthyl-5,5,8,8-tétrahydro-5,6,7,8 naphtyl-2)-4 oxo-4 méthyl-2 butyrate de sodium,
le (tétraméthyl-5,5,8,8-tétrahydro-5,6,7,8 naphtyl-2) oxo-4 butyrate de zinc,
l'acide (tétraméthyl-5,5,8,8-tétrahydro-5,6,7,8 naphtyl-2)-4 butyrique, et le (tétraméthyl-5,5,8,8-tétrahydro-5,6,7,8 naphtyl-2)-4 oxo-4 méthyl-2 butyrate de zinc.

11. Composés selon l'une quelconque des revendications 1 à 9, caractérisés par le fait qu'ils ont pris dans le groupe constitué par : l'acide (méthano-5,8 tétrahydro-5,6,7,8 naphtyl-2)-4 oxo-4 méthyl-2 butyrique,
l'acide (méthano-5,8 tétrahydro-5,6,7,8 naphtyl-2)-4 oxo-4 butyrique,
l'acide (diméthoxy-1,4 méthano-5,8 tétrahydro-5,6,7,8 naphtyl-2)-4 oxo-4 méthyl-2 butyrique,
l'acide (diméthoxy-1,4 méthano-5,8 tétrahydro-5,6,7,8 naphtyl-2)-4 oxo-4 butyrique,
le (diméthoxy-1,4 méthano-5,8 tétrahydro-5,6,7,8 naphtyl-2)-4 oxo-4 butyrate d'éthyle,

le N-éthyl (diméthoxy-1,4 méthano-5,8 tétrahydro-5,6,7,8 naphtyl-2)-4 oxo-4 butyramide,
l'acide (diméthoxy-1,4 méthano-5,8 tétrahydro-5,6,7,8 naphtyl-2)-4 hydroxy-4 butyrique, et
le (diméthoxy-1,4-méthano-5,8 tétrahydro-5,6,7,8 naphtyl-2)-4 oxo-4 butyrate de zinc.

12. Composés selon l'une quelconque des revendications 1 à 9, caractérisés par le fait qu'ils sont pris dans le groupe constitué par :
l'acide (pentaméthyl-1,1,2,3,3 indanyl-5)-4 oxo-4 méthyl-2 butyrique,
l'acide (tétraméthyl-1,1,3,3 indanyl-5)-4 oxo-4 méthyl-2 butyrique,
l'acide (pentaméthyl-1,1,2,3,3 indanyl-5)-4 oxo-4 butyrique,
l'acide (tétraméthyl-1,1,3,3 indanyl-5)-4 oxo-4 butyrique,
le (pentaméthyl-1,1,2,3,3 indanyl-5)-4 oxo-4 butyrate d'éthyle,
le N-(hydroxy-2 éthoxyéthyl)(pentaméthyl-1,1,2,3,3 indanyl-5)-4 oxo-4 butyramide,
l'acide (pentaméthyl-1,1,2,3,3 indanyl-5)-4 hydroxy-4 butyrique, et
le (pentaméthyl-1,1,2,3,3 indanyl-5)-4 oxo-4 butyrate de zinc.

13. Procédé de préparation des composés selon l'une quelconque des revendications 1 à 12 caractérisé par le fait qu'il consiste à faire réagir, dans les conditions de la réaction de Friedel-Crafts, un anhydride d'acide de formule (2)

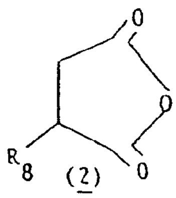

sur un composé aromatique bicyclique de formule (1)

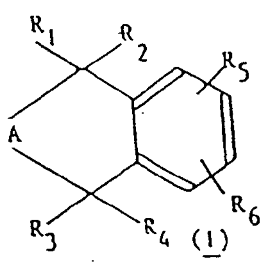

dans lesquelles :

A, $R_1$ à $R_6$ et $R_8$ ont les mêmes significations que celles données à la revendication 1, et que l'on procède, si nécessaire à partir du céto-acide obtenu aux réactions conventionnelles permettant d'accéder aux autres composés de formule (I).

14. Procédé selon la revendication 13, caractérisé par le fait que la réaction de condensation est réalisée en présence d'un acide de Lewis tel que le chlorure d'aluminium ou le chlorure d'étain, dans un solvant chloré.

15. Procédé selon la revendication 13, caractérisé par le fait que le céto-acide obtenu est réduit en hydroxyacide en présence de borohydrure de sodium dans le tétrahydrofuranne ou bien en présence de zinc en milieu alcalin.

16. Procédé selon la revendication 13, caractérisé par le fait que la fonction cétone du céto-acide obtenu est réduite à l'aide d'un amalgame de zinc en présence d'acide chlorhydrique.

17. Composition cosmétique caractérisée par le fait qu'elle contient, dans un milieu cosmétique approprié, au moins un composé de formule (I) selon l'une quelconque des revendications 1 à 12.

18. Composition cosmétique selon la revendication 17, caractérisée par le fait qu'elle contient un composé de formule (I) entre 0,005 et 5% en poids et de préférence entre 0,01 et 1% en poids.

19. Composition pharmaceutique caractérisée par le fait qu'elle contient, dans un véhicule approprié pour une administration par voie entérale, parentérale ou topique, au moins un composé de formule (I) selon l'une quelconque des revendications 1 à 12.

20. Composition selon la revendication 19, caractérisée par le fait qu'elle se présente sous une forme appropriée pour une application topique et contient de 0,01 à 10% et de préférence de 0,1 à 5% en poids d'un composé de formule (I).

21. Composition selon les revendications 17 à 20 caractérisée par le fait qu'elle contient en outre au moins un composé pharmacodynamiquement ou cosmétiquement actif tel qu'un agent hydratant, un agent anti-séborrhéique, un agent anti-acnéique, un agent antibiotique, un agent favorisant la repousse des cheveux, un agent anti-inflammatoire, un carotinoïde ou un agent anti-psoriasique.

**Patentansprüche**

1. Aromatische Buttersäure-Derivate folgender Formel:

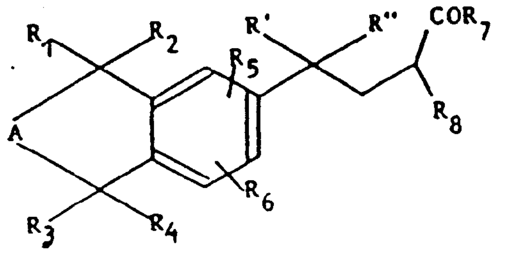

(I)

worin

$R_1$, $R_2$, $R_3$ und $R_4$ unabhängig voneinander für ein Wasserstoffatom oder einen Niedrigalkylrest stehen, wobei mindestens zwei der Reste $R_1$ bis $R_4$ kein Wasserstoffatom bedeuten;

A für einen mit einem Niedrigalkylrest substituierten oder nicht substituierten Methylen- oder Dimethylenrest steht, wobei, wenn A für einen Dimethylenrest steht, $R_1$ und $R_3$ zusammen einen Methylen- oder Dimethylenrest bilden können;

$R_5$ und $R_6$ für ein Wasserstoffatom, ein Halogenatom, einen Niedrigalkylrest, einen Niedrigalkoxyrest oder einen Hydroxylrest stehen;

R′ für ein Wasserstoffatom, einen Hydroxylrest, einen Niedrigalkoxyrest oder einen C1-$C_4$-Acyloxyrest steht;

R″ für ein Wasserstoffatom oder einen Niedrigalkoxyrest steht, oder

R′ und R″ zusammen einen Oxo-(=0)- oder Hydroxyimino(=N-OH)-rest bilden;

$R_8$ für ein Wasserstoffatom oder einen Niedrigalkylrest steht; und

$R_7$ für einen $OR_9$-Rest oder für

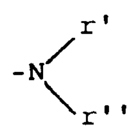

steht, wobei

$R_9$ für ein Wasserstoffatom, einen Alkylrest mit 1 bis 20 Kohlenstoffatomen, einen Monohydroxyalkyl-, Polyhydroxyalkyl-, Aryl- oder Aralkylrest, einen Zuckerrest, einen Carboxyalkyl- oder Alkoxycarbonylalkylrest steht;

r′ und r″ für ein Wasserstoffatom, einen Niedrigalkylrest, einen gegebenenfalls durch ein Heteroatom unterbrochenen Monohydroxyalkylrest, einen Polyhydroxyalkyl-, Aryl- oder Benzylrest, einen Aminosäure- oder Aminozuckerrest stehen, oder zusammen einen gegebenenfalls mit einem Hydroxy-$C_1$-$C_3$-alkylrest substituierten Heterozyklus bilden, und die Salze der Verbindungen der Formel (I) und ihre optischen Isomere sowie die tautomeren Formen der Verbindungen der Formel (I).

2. Verbindungen nach Anspruch 1, **dadurch gekennzeichnet,** daß die Niedrigalkylreste sowie die Reste mit bis zu 20 Kohlenstoffatomen ausgewählt sind unter einem Methyl-, Ethyl-, Propyl-, i-Propyl-, Butyl-, t-Butyl-, 2-Ethylhexyl-, Octyl-, Dodecyl-, Hexadecyl- und Octadecylrest.

3. Verbindungen nach Anspruch 1, **dadurch gekennzeichnet,** daß der Monohydroxyalkylrest ein 2-Hydroxyethyl-, 2-Hydroxypropyl- oder 2-Hydroxyethoxyethylrest ist.

4. Verbindungen nach Anspruch 1, **dadurch gekennzeichnet,** daß der Polyhydroxyalkylrest ein 2,3-Dihydroxypropyl-, 1,3-Dihydroxy-2-propyl- oder Pentaerythritrest ist.

5. Verbindungen nach Anspruch 1, **dadurch gekennzeichnet,** daß der Niedrigalkoxyrest ein Methoxy-, i-Propoxy-, Butoxy- oder t-Butoxyrest ist.

6. Verbindungen nach Anspruch 1, **dadurch gekennzeichnet,** daß der Zuckerrest von Glucose, Mannose, Erythrose oder Galactose abgeleitet ist.

7. Verbindungen nach Anspruch 1, **dadurch gekennzeichnet,** daß der Rest des Aminozuckers von Glucosamin, Galactosamin, Mannosamin oder Meglumin abgeleitet ist.

8. Verbindungen nach Anspruch 1, **dadurch gekennzeichnet,** daß die Reste r′ und r″ zusammen mit dem Stickstoffatom, an das sie gebunden sind, einen Piperidin-, Piperazin-, Morpholin-, Pyrrolidin- oder 4-(2-Hydroxyethyl)-piperazinrest bilden.

9. Verbindungen nach einem der vorhergehenden Ansprüche, wobei die Verbindungen folgende Formel haben:

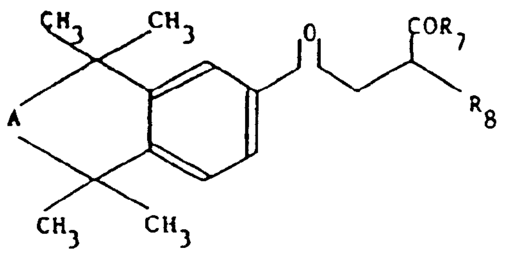

(IV)

worin

A für einen $-(CH_2)_2-$ oder

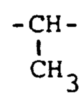

Rest steht,

$R_7$ für einen $-OR_9$-Rest oder für

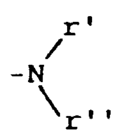

steht,

wobei $R_9$ für ein Wasserstoffatom oder einen Niedrigalkylrest steht,

r′ für ein Wasserstoffatom steht, und

r″ für einen Alkylrest mit 1 bis 8 Kohlenstoffatomen, einen gegebenenfalls mit einem Heteroatom unterbrochenen Monohydroxyalkylrest oder einen Polyhydroxyalkylrest steht, oder

r′ und r″ zusammen mit dem Stickstoffatom einen 4-(2-Hydroxyethyl)-piperazinylrest bilden, und

$R_8$ für ein Wasserstoffatom oder einen Methylrest steht.

10. Verbindungen nach einem der Ansprüche 1 bis 9, nämlich:
4-(5,5,8,8-Tetramethyl-5,6,7,8-tetrahydro-2-naphthyl)-4-oxo-2-methylbuttersäure,
4-(5,5,8,8-Tetramethyl-5,6,7,8 -tetrahydro-2-naphthyl)-4-oxobuttersäure,
Ethyl-4-(5,5,8,8,-tetramethyl-5,6,7,8-tetrahydro-2-naphthyl)-4-oxobutyrat,
2′-Ethylhexyl-4-(5,5,8,8,-tetramethyl-5,6,7,8 -tetrahydro-2-naphthyl)-4-oxobutyrat,
N-Ethyl-4-(5,5,8,8-tetramethyl-5,6,7,8-tetrahydro-2-naphthyl)-4-oxo-2-methylbutyramid,
N-Ethyl-4-(5,5,8,8-tetramethyl-5,6,7,8-tetrahydro-2-naphthyl)-4-oxobutyramid,
N-4′-(2-Hydroxyethyl)-piperazinyl-4-(5,5,8,8-tetramethyl-5,6,7,8-tetrahydro-2-naphthyl)-4-oxo-butyramid,
N-(2-Hydroxyethoxyethyl)-4-(5,5,8,8-tetramethyl-5,6,7,8-tetrahydro-2-naphthyl)-4-oxobutyramid,
4-(5,5,8,8-Tetramethyl-5,6,7,8-tetrahydro-2-naphthyl)-4-hydroxybuttersäure,
Ethyl-4-(5,5,8,8-tetramethyl-5,6,7,8-tetrahydro-2-naphthyl)-4-hydroxyiminobutyrat,
4-(5,5,8,8-Tetramethyl-5,6,7,8-tetrahydro-2-naphthyl)-4-hydroxyiminobuttersäure,
Natrium-4-(5,5,8,8-tetramethyl-5,6,7,8-tetrahydro-2-naphthyl)-4-oxobutyrat,
Natrium-4-(5,5,8,8-tetramethyl-5,6,7,8-tetrahydro-2-naphthyl)-4-oxo-2-methylbutyrat,

Zink-4-(5,5,8,8-tetramethyl-5,6,7,8-tetrahydro-2-naphthyl)-4-oxobutyrat,
4-(5,5,8,8-Tetramethyl-5,6,7,8-tetrahydro-2-naphthyl) buttersäure, und
Zink-4-(5,5,8,8-tetramethyl-5,6,7,8-tetrahydro-2-naphthyl)-4-oxo-2-methylbutyrat.

11. Verbindungen nach einem der Ansprüche 1 bis 9, nämlich:
4-(5,8-Methano-5,6,7,8-tetrahydro-2-naphthyl)-4-oxo-2-methylbuttersäure,
4-(5,8-Methano-5,6,7,8-tetrahydro-2-naphthyl)-4-oxobuttersäure,
4-(1,4-Dimethoxy-5,8-methano-5,6,7,8-tetrahydro-2-naphthyl)-4-oxo-2-methylbuttersäure,
4-(1,4-Dimethoxy-5,8-methano-5,6,7,8-tetrahydro-2-naphthyl)-4-oxobuttersäure,
Ethyl-4-(1,4-dimethoxy-5,8-methano-5,6,7,8-tetrahydro-2-naphthyl)-4-oxobutyrat,
N-Ethyl-4-(1,4-dimethoxy-5,8-methano-5,6,7,8-tetrahydro-2-naphthyl)-4-oxobutyramid,
4-(1,4-Dimethoxy-5,8-methano-5,6,7,8-tetrahydro-2-naphthyl)-4-hydroxybuttersäure, und
Zink-4-(1,4-dimethoxy-5,8-methano-5,6,7,8-tetrahydro-2-naphthyl)-4-oxobutyrat.

12. Verbindungen nach einem der Ansprüche 1 bis 9, nämlich:
4-(1,1,2,3,3-Pentamethyl-5-indanyl)-4-oxo-2-methylbuttersäure,
4-(1,1,3,3-Tetramethyl-5-indanyl)-4-oxo-2-methylbuttersäure,
4-(1,1,2,3,3-Pentamethyl-5-indanyl)-4-oxobuttersäure,
4-(1,1,3,3-Tetramethyl-5-indanyl)-4-oxobuttersäure,
Ethyl-4-(1,1,2,3,3-pentamethyl-5-indanyl)-4-oxobutyrat,
N-(2-Hydroxyethoxyethyl)-4-(1,1,2,3,3-pentamethyl-5-indanyl)-4-oxobutyramid,
4-(1,1,2,3,3-Pentamethyl-5-indanyl)-4-hydroxybuttersäure, und
Zink-(1,1,2,3,3-pentamethyl-5-indanyl)-4-oxobutyrat.

13. Verfahren zur Herstellung der Verbindungen nach einem der Ansprüche 1 bis 12, **dadurch gekennzeichnet,** daß man unter den Friedel-Crafts-Reaktionsbedingungen ein Säureanhydrid der Formel (2)

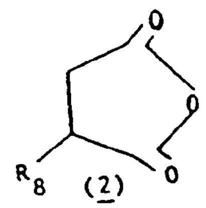

(2)

mit einer aromatischen bicyclischen Verbindung der Formel (1)

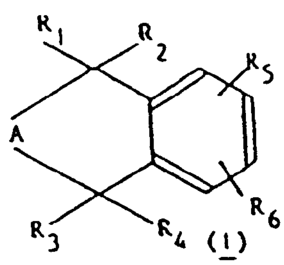

(1)

worin A, $R_1$ bis $R_6$ und $R_8$ die in Anspruch 1 angegebenen Bedeutungen besitzen, umsetzt, und wenn nötig, die erhaltene Ketosäure nach herkömmlichen Reaktionen umsetzt, welche die Herstellung der anderen Verbindungen der Formel (1) ermöglichen.

14. Verfahren nach Anspruch 13, dadurch gekennzeichnet, daß die Kondensation in Anwesenheit einer Lewis-Säure, wie Aluminiumchlorid oder Zinnchlorid, in einem chlorierten Lösungsmittel durchgeführt wird.

15. Verfahren nach Anspruch 13, dadurch gekennzeichnet, daß die erhaltene Ketosäure in Anwesenheit von Natriumborhydrid in Tetrahydrofuran oder in Anwesenheit von Zink in alkalischem Milieu zur Hydroxysäure reduziert wird.

16. Verfahren nach Anspruch 13, dadurch gekennzeichnet, daß die Keto-funktion der erhaltenen Ketosäure mit Hilfe von Zinkamalgam in Anwesenheit von Salzsäure reduziert wird.

17. Kosmetisches Mittel, dadurch gekennzeichnet, daß es in einem geeigneten kosmetischen Milieu mindestens eine Verbindung der Formel (I) nach inem der Ansprüche 1 bis 12 enthält.

18. Kosmetisches Mittel nach Anspruch 17, dadurch gekennzeichnet, daß es eine Verbindung der Formel (I) in einer Menge zwischen 0,005 und 5 Gew.% und vorzugsweise zwischen 0,01 und 1 Gew.-% enthält.

19. Kosmetisches Mittel, dadurch gekennzeichnet, daß es in einem zur enteralen, parenteralen oder topischen Verabreichung geeigneten Träger mindestens eine Verbindung der formel (I) nach einem der Ansprüche 1 bis 12 enthält.

20. Mittel nach Anspruch 19, dadurch gekennzeichnet, daß es in zur topischen Applikation geeigneter Form vorliegt und 0,01 bis 10 Gew.-% und vorzugsweise 0,1 bis 5 Gew.-% einer Verbindung der Formel (I) enthält.

21. Mittel nach den Ansprüchen 17 bis 20, dadurch gekennzeichnet, daß es außerdem mindestens eine pharmakodynamisch oder kosmetisch aktive Verbindung, wie ein Hydratisierungsmittel, ein Antiseborrhöe-Mittel, ein Anti-Akne-Mittel, ein Antibiotikum, ein Haarwuchsmittel, ein anti-inflammatorisches Mittel, ein Carotinoid oder ein Anti-Psorias-Mittel, enthält.

## Claims

1. Aromatic derivatives of butyric acid, characterized in that they correspond to the following formula:

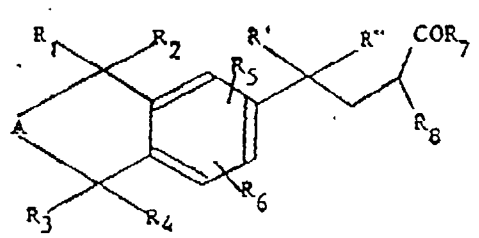

(I)

in which:

$R_1$, $R_2$, $R_3$ and $R_4$ denote, independently of one another, a hydrogen atom or a lower alkyl radical, at least two of the radicals $R_1$ to $R_4$ being other than a hydrogen atom,

A denotes a methylen or dimethylen radical, substituted or otherwise with a lower alkyl radical; when A denotes a dimethylen radical, $R_1$ and $R_3$ can form together a methylene or dimethylene radical,

$R_5$ and $R_6$ denote a hydrogen atom, a halogen atom, a lower alkyl radical, a lower alkoxy radical or a hydroxyl radical,

R′ denotes a hydrogen atom, a hydroxyl radical, a lower alkoxy radical or a $C_1$-$C_4$ acyloxy radical,

R″ denotes a hydrogen atom or a lower alkoxy radical, or R′ and R″, taken together, form an oxo (=O) or hydroxyimino (=N-OH) radical,

$R_8$ denotes a hydrogen atom or lower alkyl radical, and $R_7$ denotes a radical $-OR_9$ or

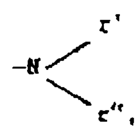

$R_9$ denoting a hydrogen atom, an alkyl radical having from 1 to 20 carbon atoms, a monohydroxyalkyl, polyhydroxyalkyl, aryl or aralkyl radical, a sugar residue or a carboxyalkyl or alkoxycarbonylalkyl radical,

r′ and r″ denoting a hydrogen atom, a lower alkyl radical, a monohydroxyalkyl radical optionally interrupted by a hetero atom, a polyhydroxyalkyl radical, an aryl or benzyl radical, or an amino acid or amino sugar residue or, taken together, form a heterocycle optionally sustituted with a $C_1$-$C_3$ hydroxyalkyl radical, and the salts of the said compounds of the formula (I) and their optical isomers as well as the tautomeric forms of the compounds of the formula (I).

2. Compounds according to Claim 1, characterised in that the lower alkyl radicals and those having up to 20 carbon atoms are selected from the group consisting of methyl, ethyl, propyl, isopropyl, butyl, tert-butyl, 2-ethylhexyl, octyl, dodecyl, hexadecyl and octadecyl radicals.

3. Compounds according to Claim 1, characterised in that the monohydroxyalkyl radical is a 2-hydroxyethyl, 2-hydroxypropyl or 2-hydroxyethoxyethyl radical.

4. Compounds according to Claim 1, characterised in that the polyhydroxyalkyl radical is a 2,3-dihydroxypropyl or 1,3-dihydroxy-2-propyl radical or a pentaerythritol residue.

5. Compounds according to Claim 1, characterised in that the lower alkoxy radical is a methoxy, isopropoxy, butoxy or tert-butoxy radical.

6. Compounds according to Claim 1, characterised in that the residue of a sugar is derived from glucose, mannose, erythrose or galactose.

7. Compounds according to Claim 1, characterised in that the amino sugar residue is derived from glucosamine, galactosamine, mannosamine or meglumine.

8. Compounds according to Claim 1, characterised in that the radicals r′ and r″, taken together, form, with the nitrogen atom to which they are attached, a piperidino, piperazino, morpholino, pyrrolidino or 4-(2-hydroxyethyl)piperazino radical.

9. Compounds according to any one of the claims, characterised in that they correspond to the following formula:

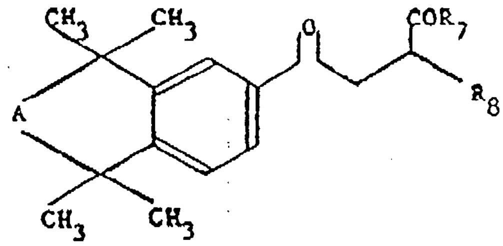

(IV)

in which:

A denotes a

$$-(CH_2)_2-$$

radical or a

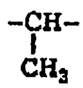

radical,

$R_7$ denotes a radical $-OR_9$ or

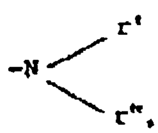

$R_9$ denoting a hydrogen atom or a lower alkyl radical,

r′ being a hydrogen atom and r″ being an alkyl radical having from 1 to 8 carbon atoms, a monohydroxyalkyl radical optionally interrupted by a hetero atom or a polyhydroxyalkyl radical, or r′ and r″, taken together, form with the nitrogen atom a 4-(2-hydroxyethyl)piperazinyl radical,

and $R_8$ denotes a hydrogen atom or a methyl radical.

10. Compounds according to any one of Claims 1 to 9, characterized in that they are selected from the group consisting of:

4-(5,5,8,8-Tetramethyl-5,6,7,8-tetrahydro-2-naphthyl)-4-oxo-2-methylbutyric acid,
4-(5,8-Methano-5,6,7,8-tetrahydro-2-naphthyl)-4-oxo-2-methylbutyric acid,
4-(1,1,2,3,3-Pentamethyl-5-indanyl)-4-oxo-2-methylbutyric acid,
4-(1,1,3,3-Tetramethyl-5-indanyl)-4-oxo-2-methylbutyric acid,
4-(5,5,8,8-Tetramethyl-5,6,7,8-tetrahydro-2-naphthyl)-4-oxobutyric acid,
4-(5,8-Methano-5,6,7,8-tetrahydro-2-naphtyl)-4-oxobutyric acid,
4-(1,1,2,3,3-Pentamethyl-5-indanyl)-4-oxobutyric acid,
4-(1,1,3,3-Tetramethyl-5-indanyl)-4-oxobutyric acid,
4-(1,4-Dimethoxy-5,8-methano-5,6,7,8-tetrahydro-2-naphtyl)-4-oxo-2-methylbutyric acid,

4-(1,4-Dimethoxy-5,8-methano-5,6,7,8-tetrahydro-2-naphtyl)-4-oxobutyric acid,
Ethyl 4-(1,1,2,3,3-pentamethyl-5-indanyl)-4-oxobutyrate,
Ethyl 4-(5,5,8,8-tetramethyl-5,6,7,8-tetrahydro-2-naphtyl)-4-oxobutyrate,
Ethyl 4-(1,4-dimethoxy-5,8-methano-5,6,7,8-tetrahydro-2-naphtyl)-4-oxobutyrate,
2′-Ethylhexyl 4-(5,5,8,8-tetramethyl-5,6,7,8-tetrahydro-2-naphthyl)-4-oxobutyrate,
N-Ethyl-4-(5,5,8,8-tetramethyl-5,6,7,8-tetrahydro-2-naphtyl)-4-oxo-2-methylbutyramide,
N-Ethyl-4-(5,5,8,8-tetramethyl-5,6,7,8-tetrahydro-2-naphtyl)-4-oxobutyramide,
N-Ethyl-4-(1,4-dimethoxy-5,8-methano-5,6,7,8-tetrahydro-2-naphtyl)-4-oxobutyramide,
N-[4′-(2-Hydroxyethyl)piperazinyl]-4-(5,5,8,8-tetramethyl-5,6,7,8-tetrahydro-2-naphthyl)-4-oxobutyramide,
N-(2-Hydroxyethoxyethyl)-4-(5,5,8,8-tetramethyl-5,6,7,8-tetrahydro-2-naphthyl)-4-oxobutyramide,
N-(2-Hydroxyethoxyethyl)-4-(1,1,2,3,3-pentamethyl-5-indanyl)-4-oxobutyramide,
4-(5,5,8,8-Tetramethyl-5,6,7,8-tetrahydro-2-naphtyl)-4-hydroxy butyric acid,
4-(1,1,2,3,3-Pentamethyl-5-indanyl)-4-hydroxybutyric acid,
4-(1,4-Dimethoxy-5,8-methano-5,6,7,8-tetrahydro-2-naphthyl)-4-hydroxybutyric acid,
Ethyl 4-(5,5,8,8-tetramethyl-5,6,7,8-tetrahydro-2-naphtyl)-4-(hydroxyimino)butyrate,
4-(5,5,8,8-Tetramethyl-5,6,7,8-tetrahydro-2-naphthyl)-4-(hydroxyimino)butyric acid,
Sodium 4-(5,5,8,8-tetramethyl-5,6,7,8-tetrahydro-2-naphthyl)-4-oxobutyrate,
Sodium 4-(5,5,8,8-tetramethyl-5,6,7,8-tetrahydro-2-naphtyl)-4-oxo-2-methylbutyrate,
Zinc (5,5,8,8-tetramethyl-5,6,7,8-tetrahydro-2-naphthyl)-4-oxobutyrate,
Zinc 4-(1,4-dimethoxy-5,8-methano-5,6,7,8-tetrahydro-2-naphthyl)-4-oxobutyrate,
Zinc (1,1,2,3,3-pentamethyl-5-indanyl)-4-oxobutyrate,
4-(5,5,8,8-Tetramethyl-5,6,7,8-tetrahydro-2-naphthyl)butyric acid, and
Zinc 4-(5,5,8,8-tetramethyl-5,6,7,8-tetrahydro-2-naphthyl)-4-oxo-2-methylbutyrate.

11. Compounds according to any one of Claims 1 to 9, characterised in that they are selected from the group consisting of:
4-(5,8-Methano-5,6,7,8-tetrahydro-2-naphtyl)-4-oxo-2-methylbutyric acid,
4-(5,8-Methano-5,6,7,8-tetrahydro-2-naphthyl)-4-oxobutyric acid,
4-(1,4-Dimethoxy-5,8-methano-5,6,7,8-tetrahydro-2-naphthyl)-4-oxo-2-methylbutyric acid,
4-(1,4-Dimethoxy-5,8-methano-5,6,7,8-tetrahydro-2-naphthyl)-4-oxobutyric acid,
Ethyl 4-(1,4-dimethoxy-5,8-methano-5,6,7,8-tetrahydro-2-napthyl)-4-oxobutyrate,
N-Ethyl-4-(1,4-dimethoxy-5,8-methano-5,6,7,8-tetrahydro-2-naphthyl)-4-oxobutyramide,
4-(1,4-Dimethoxy-5,8-methano-5,6,7,8-tetrahydro-2-naphthyl)-4-hydroxybutyric acid,
Zinc 4-(1,4-dimethoxy-5,8-methano-5,6,7,8-tetrahydro-2-naphtyl)-4-oxobutyrate,

12. Compounds according to any one of Claims 1 to 9, characterised in that they are selected from the group consisiting of:
4-(1,1,2,3,3-Pentamethyl-5-indanyl)-4-oxo-2-methylbutyric acid,
4-(1,1,3,3-Tetramethyl-5-indanyl)-4-oxo-2-methylbutyric acid,
4-(1,1,2,3,3-Pentamethyl-5-indanyl)-4-oxobutyric acid,
4-(1,1,3,3-Tetramethyl-5-indanyl)-4-oxobutyric acid,
Ethyl 4-(1,1,2,3,3-pentamethyl-5-indanyl)-4-oxobutyrate,
N-(2-Hydroxyethoxyethyl)-4-(1,1,2,3,3-pentamethyl-5-indanyl)-4-oxobutyramide,
4-(1,1,2,3,3-Pentamethyl-5-indanyl)-4-hydroxybutyric acid,
Zinc (1,1,2,3,3-pentamethyl-5-indanyl)-4-oxobutyrate,

13. Process for preparing the compounds according to any one of Claims 1 to 12, characterised in that it consists in reacting, under the conditions of the Friedel-Crafts reaction, an acid anhydride of the formula (2)

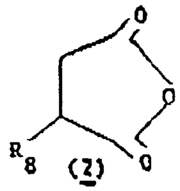

(2)

with a bicyclic aromatic compound of the formula (1)

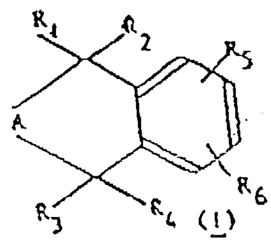

(I)

in which:

A, $R_1$ to $R_6$ and $R_6$ have the same meanings as those given in Claim 1, and in that, if necessary, using the keto acid obtained, conventional reactions enabling access to be gained to the other compounds of formula (I) are performed.

14. The process according to Claim 13, characterised in that the condensation reaction is carried out in the presence of a Lewis acid such as aluminium chloride or tin chloride, in a chlorinated solvent.

15. Process according to Claim 13, characterised in that the keto acid obtained is reduced to a hydroxy acid in the presence of sodium borohydride in tetrahydrofuran or alternatively in the presence of zinc in an alkaline medium.

16. Process according to Claim 13, characterised in that the keto group of the keto acid obtained is reduced by means of a zinc amalgam in the presence of hydrochloric acid.

17. Cosmetic composition, characterised in that it contains at least one compound of the formula (I) according to any one of Claims 1 to 12, in a suitable cosmetic medium.

18. Cosmetic composition according to Claim 17, characterised in that it contains a compound of the formula (I) at between 0.005 and 5% by weight, and preferably between 0.01 and 1% by weight.

19. Pharmaceutical composition, characterised in that it contains at least one compound of the formula (I) according to any one of Claims 1 to 12, in a vehicle suitable for an enteral, parenteral or topical administration.

20. Composition according to Claim 19, characterised in that it is presented in a form suitable for a topical application, and contains from 0.01 to 10%, and preferably from 0.1 to 5%, by weight of a compound of the formula (I).

21. Composition according to Claims 17 to 20, characterised in that it contains, in addition, at least one pharmacodynamically or cosmetically active compound, such as a moisturising agent, an antiseborrhoeic agent, an anti-acne agent, an antibiotic agent, an agent promoting hair regrowth, an anti-inflammatory agent, a carotenoid or an antipsoriatic agent.